(19) Europäisches Patentamt
European Patent Office
Office européen des brevets

(11) EP 2 177 515 A1

(12) EUROPEAN PATENT APPLICATION
published in accordance with Art. 153(4) EPC

(43) Date of publication:
**21.04.2010 Bulletin 2010/16**

(21) Application number: **08790962.8**

(22) Date of filing: **08.07.2008**

(51) Int Cl.:
***C07D 301/08*** (2006.01) ***B01J 29/89*** (2006.01)
***C07D 303/04*** (2006.01) ***C07B 61/00*** (2006.01)

(86) International application number:
**PCT/JP2008/062325**

(87) International publication number:
**WO 2009/008422 (15.01.2009 Gazette 2009/03)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.07.2007 JP 2007181969**

(71) Applicant: **Sumitomo Chemical Company, Limited**
**Tokyo 104-8260 (JP)**

(72) Inventor: **HATANO, Ryo**
**Ibaraki-shi**
**Osaka 567-0841 (JP)**

(74) Representative: **Vossius & Partner**
**Siebertstraße 4**
**81675 München (DE)**

# (54) METHOD AND APPARATUS FOR PRODUCING MIXED GAS, AND APPARATUS AND METHOD FOR PRODUCING EPOXY COMPOUND

(57) There is provided a method for producing a mixed gas which contains a combustion assisting gas and a combustible gas **characterized in that** the method includes the steps of:
(1) mixing a [combustion assisting gas and inert gas]-containing gas which contains the combustion assisting gas and an inert gas with the comestible gas so as to prepare a [combustion assisting gas, inert gas, and combustible gas]-containing gas which contains the combustion assisting gas, the inert gas, and the combustible gas; and
(2) reducing an amount of the inert gas contained in the [combustion assisting gas, inert gas, and combustible gas]-containing gas.

Such method is used for the production of a mix gas which further contains another combustible gas.

[Fig. 1]

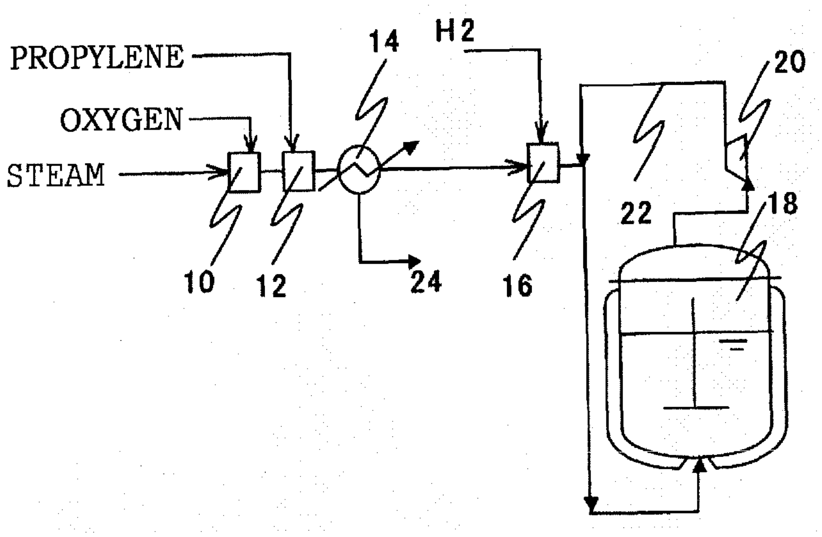


EP 2 177 515 A1

## Description

BACKGROUND OF THE INVENTION

1. Field of the Invention

The present invention relates to an apparatus and a method for producing a mixed gas containing a combustion assisting gas such as oxygen and a combustible gas such as an olefin and, further, to a method and a apparatus for producing a mixed gas containing the mixed gas and another combustible gas. For brevity, the former combustible gas will be referred to as a first combustible gas; the latter combustible gas will be referred to as a second combustible gas; the former mixed gas (i.e. the gas prepared by mixing the combustion assisting gas and the first combustible gas so as to contain the combustion assisting gas and the first combustible gas) will be referred to as a first gas; and the latter mixed gas (i.e. the gas prepared by mixing the first mixed gas and the second combustible gas so as to contain the first combustible gas and the second combustible gas) will be referred to as a second mixed gas.
Further, the present invention relates to an apparatus and a method for producing an epoxy compound corresponding to an olefin by utilizing the method and apparatus for producing the mixed gas while using the olefin as the first combustible gas.

2. Description of the Related Art

Various methods have been known for producing an epoxy compound corresponding to an olefin by using hydrogen and oxygen from the olefin such as propylene (see Japanese Patent Application Kohyo Publication Nos. 2006-517522 and 2005-514364). In such methods, hydrogen peroxide is generated by using oxygen and hydrogen for epoxidation reaction of olefin. A gas containing an oxygen gas as a combustion assisting gas and a hydrogen gas as a combustible gas has a considerably wide explosive range. Further, mixing propylene as an additional combustible gas with said gas is very difficult. Mixing of the combustion assisting gas and the combustible gas is generally carried out with an inert gas such as nitrogen. Specifically a mixed gas containing oxygen, an olefin, hydrogen and nitrogen is supplied to a reactor so as to carry out an epoxidation reaction, which is described in the above referred publications.

DICSLORURE OF THE INVENTION

1. Problem to be Solved

The inert gas is not substantially involved in the reaction, exits from a reaction system together with reaction products, and it is to be ultimately discharged as an exhaust gas. Such an exhaust gas contains valuable components (e.g. propylene, propylene oxide, etc.) which are accompanied with the exhaust gas. Therefore, it is necessary to recover the valuable components. Further, the inert gas which is not involved in the reaction occupies an major amount of the reactor volume, so that there is a problem in that such inert gas reduces an epoxidation reaction rate.

2. Means to Solve the Problem

As a result of extensive research of the inventor on the problems, the inventor has found that a gas which comprises a combustion assisting gas and a combustible gas is obtained by:

mixing, preferably sufficiently uniformly mixing a [combustion assisting gas and inert gas]-containing gas which comprises the combustion assisting gas and the inert gas, preferably in a sufficiently uniformly mixed state with a combustible gas (corresponding to the first combustible gas) so as to prepare a [combustion assisting gas, inert gas, and combustible gas]-containing gas (corresponding to the first mixed gas) which comprises the combustion assisting gas, the inert gas, and the combustible gas; and
reducing an amount of the inert gas from the prepared gas, and thereby the object of the present invention is attained.

Particularly, in the case of using water vapor (or steam) as the inert gas, the following have been found:

the water vapor acts as a diluting agent like nitrogen gas with respect to explosion (or combustion) of the combustible gas in the presence of the combustion assisting gas; and,
from a mixed gas containing a combustible gas such as an olefin and hydrogen, a combustion assisting gas such as oxygen, and water vapor, it is possible to obtain a mixed gas containing the combustible gas, the combustion assisting gas, and water vapor with a reduce amount of water vapor by cooling and/or compressing the mixed gas,

preferably such mixed gas of which amount of water vapor corresponds to a saturated water vapor pressure of conditions for the cooling and/or compression. The above features have been found to be useful for a reaction of a combustible gas and a gas containing oxygen, such as a method for producing, from an olefin, an epoxy compound corresponding to the olefin by reacting with hydrogen and oxygen.

According to the first aspect, the present invention provides a method for producing a mixed gas containing a combustion assisting gas and a combustible gas (corresponding to a first combustible gas), which method comprises:

(1) the step of mixing a [combustion assisting gas and inert gas]-containing gas which comprises the combustion assisting gas and an inert gas with the comestible gas so as to prepare a [combustion assisting gas, inert gas, and combustible gas]-containing gas which comprises the combustion assisting gas, the inert gas, and the combustible gas; and
(2) the step of reducing an amount of the inert gas contained in the [combustion assisting gas, inert gas, and combustible gas]-containing gas.

The above mentioned mixing step (1) may be referred to as a first mixing step in connection with a second mixing step which will be described below. The above mentioned reducing step (2) prepares a [combustion assisting gas, inert gas, and combustible gas]-containing gas (corresponding to a first mixed gas) with a reduced amount of the inert gas which comprises the combustion assisting gas, the inert gas and the combustible gas as the mixed gas containing the combustion assisting gas and the combustible gas.

In addition, the present invention provides an apparatus for producing a mixed gas (corresponding to a first mixed gas) containing a combustion assisting gas and a combustible gas (corresponding to a first combustible gas); which apparatus comprising:

(A) a mixing apparatus for mixing the combustion assisting gas, an inert gas and the combustible gas which comprises:

a means for supplying a [combustion assisting gas and inert gas]-containing gas which comprises the combustion assisting gas and the inert gas;
a means for supplying the comestible gas; and
a means for mixing those gases to prepare a [combustion assisting gas, inert gas, and combustible gas]-containing gas; and

(B) an apparatus for reducing an amount of the inert gas of the [combustion assisting gas, inert gas, and combustible gas]-containing gas which is supplied from the mixing apparatus (A).

The means for supplying the gas is not particularly limited one as far as it is able to supply the intended gas. For example, such means includes a line like a pipeline and a related ancillary equipment. Further, the means for mixing the gases is not particularly limited as far as it is able to mix the intended gases. For example, various types of stirrers may be used as such means. In addition, the apparatus for reducing an amount of the inert gas is not particularly limited as far as it is able to reduce an amount of the inert gas. For example, a mechanism which condenses, or adsorbs the inert gas.

The above mentioned mixing apparatus corresponds to a first mixing apparatus for mixing the [combustion assisting gas and inert gas]-containing gas which comprising the combustion assisting gas and an inert gas with the comestible gas so as to prepare the [combustion assisting gas, inert gas, and combustible gas]-containing gas which comprises the combustion assisting gas, the inert gas, and the combustible gas.

The above mentioned apparatus for reducing reduces an amount of the inert gas contained in the [combustion assisting gas, inert gas, and combustible gas]-containing gas so as to prepare a [combustion assisting gas, inert gas, and combustible gas]-containing gas of which inert gas amount is reduced (corresponding to a first mixed gas) as the mixed gas containing the combustion assisting gas and the combustible gas.

According to a second aspect, the present invention provides a method for producing a [combustion assisting gas, inert gas, first combustible gas and second combustible gas]-containing gas (corresponding to a second mixed gas) which comprises the combustion assisting gas, the inert gas, and a first combustible gas and a second combustible gas by a second mixing step (3) for mixing the second combustible gas as a different combustible gas with the [combustion assisting gas, inert gas and first combustible gas]-containing gas with the reduce amount of the inert gas that is obtained by the above-described method or apparatus for producing the first mixed gas of the present invention which contains as the combustible gas the first combustible gas.

In addition, the present invention provides an apparatus for producing a mixed gas (corresponding to a second mixed gas) containing the combustion assisting gas, the combustible gas (corresponding to a first combustible gas),

and a different combustible gas, which apparatus comprising:

(C) a second mixing apparatus in addition to (A) the mixing apparatus for mixing as a first mixing apparatus and (B) the apparatus for reducing an amount of the inert gas of the above-described apparatus for producing the first mixed gas of the present invention, the second mixing apparatus comprising:

a means for supplying the [combustion assisting gas, inert gas and first combustible gas]-containing gas which is obtained in the apparatus for reducing an amount of the inert gas;
a means for supplying a second combustible gas as the different combustible gas; and
a means for mixing those gases.

The second mixing apparatus prepares the [combustion assisting gas, inert gas, first combustible gas and second combustible gas]-containing gas by mixing the [combustion assisting gas and first combustible gas]-containing gas (corresponding to the first mixed gas) with reduced inert gas amount with the second combustible gas as the different combustible gas so as to prepare the [combustion assisting gas, inert gas, first combustible gas, and second combustible gas]-containing gas (corresponding to a second mixed gas) as the mixed gas containing the combustion assisting gas, the inert gas, the first combustible gas, and the second combustible gas.

In the present invention, the first and second mixing steps are not particularly limited insofar as the mixing steps are capable of mixing, preferably sufficiently uniformly mixing, the gases to be mixed, and it is possible to employ a mixing process using an ordinary gas mixing apparatus. Various kinds of mixers may be used, and for example, a T-form tube mixer, a Y-form tube mixer, a static mixer having a mixing structure such bubbles in a flow passage, and a mixer having a stirrer may be used. Such apparatus may form the production methods and also the production apparatus according to the present invention.

In the present invention, the inert gas reduction step is not particularly limited insofar as the inert gas reduction step is capable of reducing an amount of the gas to be reduced, preferably as much as possible, and it is possible to select a conventional method depending on the gas to be reduced.

For example, a condenser, an adsorber or an adsorption column may be used. When the inert gas is water vapor (or steam), it is preferable to use the condenser. Such apparatus is capable of forming the inert gas reduction apparatus in the production apparatus of the present invention. The reduction of the inert gas is conducted depending on application of the mixed gas to be prepared, and up to the extent at which the amount of the inert gas in the mixed gas is within the acceptable range.

In the present invention, the combustion assisting gas is oxygen or a gas containing oxygen (e.g. air); the combustible gas is a gas that attains combustion by the combustion assisting gas, such as an alkane, an alkene, hydrogen and the like; and the inert gas is a gas capable of forming a region out of the explosion range in the three component-system formed of the inert gas, the combustion assisting gas, and the combustible gas, such as water vapor (or steam) and carbon dioxide. According to a particularly preferable embodiment, the combustion assisting gas is oxygen, the inert gas is water vapor, the first combustible gas is an alkene such as an $\alpha$-olefin, for example, propylene, and the second combustible gas is hydrogen.

Therefore, a method for producing a mixed gas containing oxygen and an olefin (e.g. an $\alpha$-olefin such as propylene) according to the present invention includes:

(1) a first mixing step for mixing an [oxygen and water vapor]-containing gas which comprises oxygen and water vapor with the olefin so as to prepare an [oxygen, water vapor, and olefin]-containing gas which comprised oxygen, water vapor, and the olefin; and (2) a water vapor reduction step for reduction an amount of water vapor contained in the [oxygen, water vapor, and olefin]-containing gas so as to prepare an [oxygen, water vapor, and olefin]-containing gas (corresponding to the first mixed gas) of which amount of water vapor is reduced as the mixed gas containing oxygen and the olefin.

In addition, the production method of the present invention for producing a mixed gas containing oxygen, an olefin (e.g. an $\alpha$-olefin such as propylene), and hydrogen comprises:

(1) a first mixing step for mixing an [oxygen and water vapor]-containing gas which comprises oxygen and water vapor with the olefin so as to prepare an [oxygen, water vapor, and olefin]-containing gas which comprises oxygen, water vapor, and the olefin;
(2) a water vapor reduction step for reducing an amount of water vapor contained in the [oxygen, water vapor, and olefin]-containing gas so as to prepare an [oxygen, water vapor, and olefin]-containing gas (corresponding to the first mixed gas) of which amount of water vapor is reduced as a mixed gas which contains oxygen and olefin; and
(3) a second mixing step for mixing the [oxygen, water vapor, and olefin]-containing gas of which amount of water

vapor is reduced with hydrogen so as to prepare an [oxygen, water vapor, olefin, and hydrogen]-containing gas (corresponding to the second mixed gas) as the mixed gas containing oxygen, the olefin, and hydrogen.

In the water vapor reduction step, it is possible to reduce an amount of water vapor by a condensing step for condensing water vapor to remove liquefied moisture, an adsorbing step for causing an adsorbing agent to adsorb water vapor for reducing water vapor, or the like, and the condensing step is particularly preferred.

Further, an apparatus of the present invention for producing a mixed gas containing oxygen, and an olefin includes:

(A) a first mixing apparatus for mixing an [oxygen and water vapor]-containing gas which comprises oxygen and water vapor with the olefin so as to prepare an [oxygen, water vapor, and olefin]-containing gas which comprises oxygen, water vapor, and the olefin; and,
(B) a water vapor reduction apparatus for reducing an amount of water vapor contained in the [oxygen, water vapor, and olefin]-containing gas so as to prepare an [oxygen, water vapor, and olefin]-containing gas (corresponding to the first mixed gas) of which amount of water vapor is reduced.

In addition, an apparatus for producing a mixed gas containing oxygen, an olefin, and hydrogen of the present invention comprises:

(A) a first mixing apparatus for mixing the [oxygen and water vapor] [-containing gas which comprises oxygen and water vapor with the olefin so as to prepare an [oxygen, water vapor, and olefin]-containing gas which comprises oxygen, water vapor, and the olefin;
(B) a water vapor reduction apparatus for reducing an amount of water vapor contained in the [oxygen, water vapor, and olefin]-containing gas so as to prepare an [oxygen, water vapor, and olefin]-containing gas (corresponding to the first mixed gas) of which amount of water vapor is reduced; and

(3) a second mixing apparatus for mixing the [oxygen, water vapor, and olefin]-containing gas of which amount of water vapor is reduced with hydrogen so as to prepare an [oxygen, water vapor, olefin, and hydrogen]-containing gas (corresponding to the second mixed gas) which comprises oxygen, water vapor, the olefin, and hydrogen.
The above mentioned first and second mixing apparatuses comprises a means for supplying a gas to be mixed, and the above mentioned water vapor reduction apparatus comprises a means to reduce an amount of water vapor. As far as an amount of water vapor is reduced, the means may be any appropriate one.

As the water vapor reduction apparatus, it is possible to use a condenser, an adsorbing device, an adsorbing column, or the like, and the condenser is particularly preferred. The production method and the production apparatus of the present invention are suitable for continuously producing the mixed gas.

According to a third aspect, the present invention provides a method for producing an epoxy compound of an olefin, the method comprising:

(a) a step for obtaining, by the above-described method for producing the second mixed gas, an [oxygen, water vapor, olefin, and hydrogen]-containing gas (corresponding to the second mixed gas);
(b) a step for supplying the obtained [oxygen, water vapor, olefin, and hydrogen]-containing gas to a reactor; and
(c) a step for reacting oxygen, the olefin, and hydrogen in the presence of a catalyst in the reactor so as to prepare the epoxy compound corresponding to the olefin.

In addition, the present invention provides an apparatus for epoxidizing an olefin, comprising the second mixed gas production apparatus of the present invention and a liquid phase reactor, wherein the [oxygen, water vapor, olefin, and hydrogen]-containing gas (corresponding to the second mixed gas) obtained by the second mixed gas production apparatus is supplied to the reactor; and the reactor reacts oxygen, the olefin, and hydrogen in the presence of a catalyst in a liquid phase so as to prepare an epoxy compound corresponding to the olefins.

According to a fourth aspect, the present invention provides another method for producing an epoxy compound of an olefin, the method comprising:

(1) a step for obtaining, by the above-described method for producing the first mixed gas of the present invention, an [oxygen, water vapor, and olefin]-containing gas (corresponding to the first mixed gas);
(2) a step for supplying the obtained [oxygen, water vapor, and olefin]-containing gas and hydrogen together or separately to a reactor; and
(3) a step for reacting oxygen, the olefin, and hydrogen in the reactor in the presence of a catalyst in a liquid phase

to prepare the epoxy compound corresponding to the olefin.

The present invention provides another apparatus for epoxidizing an olefin, comprising the first mixed gas production apparatus of the present invention and a liquid phase reactor, wherein the [oxygen, water vapor, and olefin]-containing gas (corresponding to the first mixed gas) obtained by the first mixed gas production apparatus is supplied to the reactor; hydrogen is supplied to the reactor; and the reactor reacts oxygen, the olefin, and hydrogen in the presence of a catalyst in a liquid phase so as to prepare an epoxy compound corresponding to the olefin. In the above mentioned apparatus, the reactor comprises a means to supply hydrogen.

In any of the above-described methods and apparatuses for producing the epoxy compound, the olefin may preferably be an $\alpha$-olefin, particularly propylene. Such production methods and production apparatuses are suitable for the method for continuously producing the epoxy compound.

Effect of the Invention

According to the methods for the production of the mixed gases, the combustion assisting gas and the combustible gas(es) are safely mixed. Further, the mixed gas prepared by the apparatus according to the present invention is a mixed gas of the combustion assisting gas and the combustible gas of which inert gas amount is reduced.

BRIEF DESCRIPTION OF THE DRAWINGS

Fig. 1 is a flow sheet of a process for producing an epoxy compound of the present invention, wherein a method or apparatus for producing a mixed gas of the present invention is used in combination with an epoxidation reaction;
Fig. 2 is a flow sheet of another process for producing an epoxy compound of the present invention, wherein a method or apparatus for producing a mixed gas of the present invention is used in combination with an epoxidation reaction;
Fig. 3 is a flow sheet of a further process for producing an epoxy compound of the present invention, wherein a method or apparatus for producing a mixed gas of the present invention is used in combination with an epoxidation reaction;
Fig. 4 is an equilateral triangle coordinate graph schematically showing an explosion range of a combustion assisting gas-inert gas-combustible gas system;
Fig. 5 is an equilateral triangle coordinate graph schematically showing an explosion range of a combustion assisting gas-first combustible gas-second combustible gas system;
Fig. 6 is an equilateral triangle coordinate graph showing a result of estimation of an explosion range of an oxygen-water vapor-propylene system; and
Fig. 7 is an equilateral triangle coordinate graph showing a result of estimation of an explosion range of an oxygen-hydrogen-propylene system.

In the drawings, the reference numbers indicate the following members:
10 - premixing apparatus, 12 - first mixing apparatus, 14 - condenser, 16 - second mixing apparatus, 18 - expoxidation reactor, 20 - compressor, 22 - outside circulation line, 24 - drain, 26 - ejector, 28 - gas-liquid separation drum

DETAILED DESCRIPTION OF THE INVENTION

The apparatuses and the methods for producing the mixed gas according to the present invention as well as the apparatuses and the methods for producing the epoxy compound according to the present invention use the [combustion assisting gas and inert gas]-containing gas which contains the combustion assisting gas and the inert gas. Therefore, a description relating to the use of such [combustion assisting gas and inert gas]-containing gas in this specification is applicable to each of the apparatuses and the methods for producing the mixed gas as well as the apparatuses and the methods for producing the epoxy compound of the present invention within an applicable extent.

The inert gas (e.g. water vapor) is reduced in the apparatuses and the methods for producing the mixed gas. Though the condensation operation or the adsorption operation as described above may be preferably performed for the reduction, these operations may be performed in combination.

In the present invention, a plurality of the combustible gases, for example hydrogen and an olefin may be used. When two combustible gases are used, they may be referred to as a first combustible gas and a second combustible gas. Since it is particularly preferable to use water vapor as the inert gas, oxygen as the combustion assisting gas, propylene as the first combustible gas, and hydrogen as the second combustible gas, as well as the condensation

operation for the reduction of the inert gas, using water vapor, oxygen, the olefin (or propylene), and hydrogen as well as the condensation operation will be used as an example for explaining the present invention in description of this specification, particularly in the following description.

However, based on the concept of the present invention that it is possible to more safely prepare a mixture of the combustion assisting gas and the combustible gas by diluting the combustion assisting gas with the use of the inert gas and then reducing an amount of the inert gas, those skilled in the art can easily arrive at the production of a mixed gas using an inert gas other than water vapor (for example carbon dioxide), a combustion assisting gas other than oxygen (for example air), a combustible gas(es) other than propylene and hydrogen, and an inert gas reduction operation other than the condensation operation (for example adsorption operation) based on the disclosure of this specification. Other inert gases, combustible assisting gases, and inert gas reduction operations will be described as required in addition to water vapor, oxygen, and the condensation operation.

The reduction of water vapor by way of the condensation operation may preferably be performed in such a manner as to remove water vapor up to a degree of saturated vapor pressure under the condensation conditions, and the reduction of water vapor by way of the adsorption operation may be preferably performed in such a manner as to remove 95% or more of water vapor. Condensers such as various heat exchangers may be used in the condensation operation as described later in this specification, and an adsorption agent such as a molecular sieve may be used in the adsorption operation.

According to one embodiment of the present invention,

(1) a first mixing step is carried out in a first mixing apparatus for mixing an [oxygen and water vapor]-containing gas which comprises oxygen and water vapor with an olefin so as to prepare an [oxygen, water vapor, and olefin]-containing gas which comprises oxygen, water vapor, and the olefin;
(2) a condensing step is carried out by a condenser for reducing an amount of water vapor contained in the [oxygen, water vapor, and olefin]-containing gas so as to prepare an [oxygen, water vapor, and olefin]-containing gas (corresponding to the first mixed gas) of which amount of water vapor is reduced as a mixed gas which comprises oxygen and the olefin; and
(3) a second mixing step is carried out in a second mixing apparatus for mixing the [oxygen, water vapor, and olefin]-containing gas of which amount of water vapor is reduced with hydrogen so as to prepare an [oxygen, water vapor, olefin, and hydrogen]-containing gas (corresponding to the second mixed gas) as a mixed gas which comprises oxygen, water vapor, the olefin, and hydrogen.

It is noted that the amount of water vapor contained in the [oxygen, water vapor, and olefin]-containing gas obtained by the condensing step (or the condenser) is preferably substantially equivalent to an amount which corresponds to a saturated water vapor pressure under the conditions of the condensing step, and, in this case, such an amount of water vapor is contained in the [oxygen, water vapor, and olefin]-containing gas obtained in the second mixing step (or the second mixing apparatus).

In this case, the first mixing apparatus and the second mixing apparatus are not particularly limited insofar as they have a function of mixing gases, and it is preferable to use those capable of uniformly mixing as rapid as possible. Specifically, various mixers are usable. For example, a T-form tube mixer, a Y-form tube mixer, a static mixer such as a mixer having a structural body such as a baffle in a flow passage, a stirring blade type mixer, and the like are usable.

In addition, the condenser is not particularly limited insofar as the condenser is capable of condensing water vapor contained in the [oxygen, water vapor, and olefin]-containing gas and separating water obtained by such condensing (containing a dissolved olefin and, of course, dissolved oxygen). Specifically, various heat exchangers are usable. In this case, a gas-liquid separation vessel capable of separating and removing the water generated by the condensation may be preferably connected to the heat exchanger. A part of the water generated by the condensation may be preferably recovered as water vapor which is evaporated at a pressure lower than that of the condensation conditions. In this case, as a heat source required for the evaporation, it is preferable to use a latent heat generated when condensing water vapor contained in the [oxygen, water vapor, and olefin]-containing gas. For example, the water vapor of the [oxygen, water vapor, and olefin]-containing gas is condensed at a higher temperature side of the heat exchanger used as the condenser, while the water obtained by the condensation is heated at a lower temperature side so as to be evaporated. In another embodiment, it is possible to perform more efficient heat exchange by supplying the water in a flush drum or the like so as to boil the water, which is then supplied to a lower side of the heat exchanger.

Therefore, in the mixed gas production apparatus of the present invention, the condenser includes the heat exchanger and the gas-liquid separator, wherein the heat exchanger is provided with a coolant flow passage at a lower side and a gas flow passage at a higher temperature side; the [oxygen, water vapor, and olefin]-containing gas is flown through the gas flow passage to condensate water vapor; a gas-liquid mixture fluid after the condensation flows into the gas-liquid separator to be separated into a gas and a liquid; and, when so required, the liquid is flushed at a lower pressure , followed by being supplied to the coolant flow passage of the heat exchanger.

According one embodiment of the present invention, a preliminary mixing step for preparing an [oxygen and water vapor]-containing gas by mixing oxygen and water vapor may be preferably performed in a third mixing apparatus. The descriptions relating the first mixing apparatus or the second mixing apparatus are applicable to this mixing apparatus.

In the present invention, the [oxygen and water vapor]-containing gas means a mixed gas comprising oxygen and water vapor as described above. A ratio between oxygen and water vapor is adjusted by controlling a mixing ratio between the [oxygen and water vapor]-containing gas and the olefin in such a manner that an oxygen concentration in the [oxygen, water vapor, and olefin]-containing gas obtained by mixing the [oxygen and water vapor]-containing gas with the olefin is outside an explosion range, and generally lower than a critical oxygen concentration (or critical oxygen amount).

In a broader concept, this means that the mixing of the [combustion assisting gas and inert gas]-containing gas with the combustible gas performed in the mixing step is carried out by adjusting a composition and/or an amount of the [combustion assisting gas and inert gas]-containing gas to be mixed as well as an amount of the combustible gas to be mixed in such a manner that a composition of the [combustion assisting gas, inert gas, and combustible gas]-containing gas to be obtained after the mixing of the [combustion assisting gas and inert gas]-containing gas with the combustible gas is positioned outside the explosion range of a combustion assisting gas-inert gas-combustible gas system.

In general, the explosion range is narrowed with an increase in an amount of the inert gas to be added to the combustible gas in the coexistence of oxygen, and the explosion range disappears when the added amount of the inert gas exceed a certain amount. The concentration of oxygen upon such disappearance is called the limit oxygen concentration, and the effect of narrowing the explosion range becomes remarkable with an increase of a specific heat of the inert gas (see Literature 1: Safety Technology of Chemical Substances (Kagaku-bussitsu no Anzen-gijutsu (in Japanese)); Tadao Yoshida, and Tei Taigyoku; Tokyo Progress System; November 1, 1996; pp. 112-113).

As to the explosion range of the combustible gas, water vapor is known to act as an inert gas like nitrogen. Therefore, with respect to the oxygen-water vapor-olefin system to which the present invention is applied, in the case of adding water vapor to an olefin in the presence of oxygen, when a ratio of water vapor exceeds a certain value (i.e. when a ratio of oxygen in this gas becomes lower than a certain value), the composition of the [oxygen, water vapor, and olefin]-containing gas departs outside from the explosion range, and the ratio of the oxygen at such departure is the limit oxygen concentration. It is preferable to adjust the composition (an amount ratio between water vapor and oxygen) and the amount of the [oxygen and water vapor]-containing gas as well as the amount of the olefin to be mixed which are sued in the first mixing step (or in the first mixing apparatus) in such a manner that the oxygen concentration in the [oxygen, water vapor, and olefin]-containing gas is equal to or lower than the limit oxygen concentration. It is possible to experimentally detect such limit oxygen concentration by using an explosion range measurement device, or it is possible to use limit oxygen concentrations described in various literatures (such as Literature 1 and Literature 2: New Safety Engineering Handbook (Shin-anzen-kougaku Binran (in Japanese)); edited by Safety Engineering Association (Anzen-kougaku Kyoukai in Japanese); Colona Publishing Company; July 20, 1999; Newest Version; for example PP. 140-150), and, further, it is possible to detect the limit oxygen concentration by using experimentally, experientially, and/or theoretically established features in combination with the above.

According to a more preferred embodiment, in addition to that the oxygen concentration in the ultimately obtained [oxygen, water vapor, and olefin]-containing gas is equal to or lower than the limit oxygen concentration as described above, it is preferable that the composition (an amount ratio between water vapor and oxygen) and the amount of the [oxygen and water vapor]-containing gas as well as the amount of the olefin to be mixed with the [oxygen and water vapor]-containing gas which are used in the first mixing step (or mixed in the first mixer) are adjusted so that the oxygen concentration in the [oxygen, water vapor, and olefin]-containing gas during a course of the reduction of water vapor is continuously equal to or lower than the limit oxygen concentration.

In other words using a broader concept, the ratio between the combustion assisting gas and the inert gas contained in the [combustion assisting gas and inert gas]-containing gas to be mixed with the combustible gas in the first mixing step (1) is selected so that, in an equilateral triangle coordinate graph indicating the explosion range of the combustion assisting gas-inert gas-combustible gas system, a combustible gas addition line connecting a point corresponding to a combustion assisting gas concentration in the [combustion assisting gas and inert gas]-containing gas (provided that only the amounts of the combustion assisting gas and the inert gas are considered, and an amounts of the other component(s) is not considered when such other component(s) is present in addition to the combustion assisting gas and the inert gas) and an apex of 0% of the combustion assisting gas, 0% of the inert gas, and 100% of the combustible gas does not intersect the explosion range of the combustion assisting gas-inert gas-combustible gas system.

The equilateral triangle coordinate graph indicating the explosion range of the combustion assisting gas-inert gas-combustible gas (corresponding to the first combustible gas) system is schematically shown in Fig. 4. Such graph is obtained according to the literatures or experimentally as to the explosion range of the three-component system.
An apex A corresponds to a composition wherein the combustion assisting gas (e.g. oxygen) is 100%; an apex B corresponds to a composition wherein the inert gas (e.g. water vapor) is 100 %; and an apex C corresponds to a

composition wherein the combustible gas is 100%. An area enclosed by an oblique side AC of the equilateral triangle and a curve GH indicated with shading is the explosion range.

With the method for producing the mixed gas of the present invention, a point D on the oblique side AB of the equilateral triangle in Fig. 4 represents a composition of the [combustion assisting gas and inert gas]-containing gas to be mixed with the combustible gas. In the equilateral triangle coordinate graph, when the combustible gas is gradually added to the [combustion assisting gas and inert gas]-containing gas and the gases are mixed, a composition after the mixing moves toward the apex C from the point D along a straight line (corresponding to the above-described combustible gas addition line) connecting the apex C and the point D and ultimately reaches a composition of a point E when a predetermined amount of the combustible gas is mixed. In this case, it is preferable that the composition E that is ultimately reached composition when the combustible gas is added is outside the explosion range.

In the case where the straight line connecting the apex C and the point D passes through the explosion range indicated with shading, there is a risk of explosion of the mixed gas. Therefore, in a more preferred embodiment, it is preferable to select the point D in such a manner that the combustible gas addition line does not pass through the explosion range.

According to one preferred embodiment of the present invention, the olefin is an $\alpha$-olefin, particularly propylene, and the inventor has concluded that it is appropriate to use the data of the literatures as described below with respect to a system in which propylene is present. For example, it is described in literature 1 (see p. 113, Table 11.2) that a limit oxygen concentration of a mixed gas consisting of oxygen, propylene, and nitrogen is 11.5 vol%. The value of the limit oxygen concentration has been applied to the [oxygen, water vapor, and propylene]-containing gas in the present invention. Validity of such application will be confirmed by the following idea.

The value of the literature (11.5 vol%) is the value in the propylene-oxygen-nitrogen system. Comparing the specific heat of the nitrogen gas and that of water vapor at a temperature of 400 K and a pressure of 1 atm, the specific feat of water vapor is 36.21 J/K/mol while that of nitrogen is 29.27 J/K/mol. Therefore, the specific heat of water vapor is larger, and therefore water vapor has a greater effect of narrowing the explosion range as compared to nitrogen. In view of this, the explosion range of the system in which water vapor is present in place of nitrogen is considered to be narrower than the explosion range when nitrogen is present. That is, it is possible to estimate without difficulty that the system in which water vapor is present has a larger limit oxygen concentration. Therefore, it is safer in terms of possibility of explosion to apply the value of the literature of the system as it is in which nitrogen is present to the system in which water vapor is present in place of nitrogen, and, consequently, it is reasonable to use the value of 11.5 vol% as the value of the limit oxygen concentration of the [oxygen, water vapor, and propylene]-containing gas from the practical point of view.

As another discussion, it is possible to estimate the explosion range by using a value(s) of literature and a feature(s) experientially confirmed in combination as described below:

> By using the limit oxygen concentration of 11.5 vol% of the mixed gas consisting of oxygen, propylene, and nitrogen, the values of literatures of the explosion upper limit concentration and the explosion lower limit concentration of propylene in the air (2.4% and 11% respectively) (see the above literature 1 (see p. 111 and Table 11.1)), it is possible to estimate an explosion range of the olefin-oxygen-water vapor system, and the explosion range is shown in an equilateral triangle coordinate graph in Fig. 6.

In general, the explosion lower limit concentration is substantially constant irrespective of an oxygen concentration, an explosion lower limit concentration under an arbitrary oxygen concentration is equal to the propylene explosion lower limit concentration in the air (o in Fig. 6), and thus it is possible to draw a horizontal line with respect to the oxygen axis. By drawing a line passing through the propylene upper limit concentration in oxygen ($\Delta$ in Fig. 6) from a limit oxygen concentration position on the horizontal line (♦ in Fig. 6), it is possible to estimate the explosion range of the present system. In addition, since water vapor generally has a greater effect of narrowing the explosion range as compared to nitrogen (see literature 1, pp. 112-113), it is considered that it is possible to ensure safety in practice with the use of explosion range data obtained in a nitrogen containing system. In Fig. 6, the range enclosed by two lines indicating the explosion limit and a left oblique side of the equilateral triangle is the estimated explosion range.

Therefore, according to one embodiment of the present invention, the composition (an amount ratio between water vapor and oxygen) and the amount of the [oxygen and water vapor]-containing gas as well as the amount of propylene to be mixed therewith are adjusted in such a manner that the oxygen concentration in the [oxygen, water vapor, and propylene]-containing gas is preferably less than 11.5 vol%, more preferably 10 vol% or less, particularly preferably from 3 to 9.5 vol%, for example 8 vol%. For example, according to one particularly preferred embodiment, the [oxygen and water vapor]-containing gas contains 11.5 vol% of water vapor, and when propylene is added to such a gas, the oxygen concentration in the [oxygen, water vapor, and propylene]-containing gas is naturally reduced to less than 11.5 vol%. Such an embodiment is desirable from the safety view point.

In the present invention, it is preferable to select the composition of oxygen and the olefin in the [oxygen, water vapor, and olefin]-containing gas with a reduced amount of water vapor that is obtained in the case of mixing the [oxygen

and water vapor]-containing gas with the olefin and then reducing water vapor by the condensation in the condensing step (or in the condenser) in such a manner that the composition is outside the explosion range. It is noted that the [oxygen, water vapor, and olefin]-containing gas after the condensation contains an amount of water vapor corresponding to the saturated vapor pressure under the condensation conditions, and such amount can be ignored since the amount is small as compared to amounts of the other gases.

In other words using a broader concept, in the mixing step (1), the amount of the combustible gas to be mixed with the [combustion assisting gas and inert gas]-containing gas is selected in such a manner that an inert gas reduction line connecting the point corresponding to a combustible gas concentration in the mixed gas to be produced (provided that only the amounts of the combustion assisting gas and the combustible gas are considered, and amount(s) of the other component(s) is not considered in the case where the other component(s) is present in addition to the combustion assisting gas and the combustible gas) and an apex of 0% of the combustion assisting gas, 100% of the inert gas, and 0% of the combustible gas does not intersect with the explosion range of the combustion assisting gas-inert gas-combustible gas system.

With reference to Fig. 4, in the method for producing the mixed gas of the present invention, after the [combustion assisting gas, inert gas, and combustible gas]-containing gas having a composition E is prepared by mixing with the combustible gas, an amount of the inert gas is reduced. In the reduction step, the gas composition moves toward a point F from the point E on a straight line BF(corresponding to an inert gas reduction line) connecting the point E and the apex B to give the combustion assisting gas, inert gas, so that a [combustion assisting gas, inert gas, and combustible gas]-containing gas is obtained of which amount of the inert gas is reduced. Since it is preferable that the amount of the inert gas contained therein is as small as possible in practice, it can be considered that the composition of the gas after reducing the inert gas corresponds to the point F.

In actuality, it is necessary to maintain proportions of the combustion assisting gas and the combustible gas contained in the [combustion assisting gas, inert gas, and combustible gas]-containing gas of which amount of the inert gas is reduced at predetermined values in accordance with the usage of the [combustion assisting gas, inert gas, and combustible gas]-containing gas (therefore, it is required for a certain point on the oblique side AC of the equilateral triangle to be a point of the predetermined proportion, such as a proportion of the point F). Consequently, the point F is fixed as the predetermined composition on the oblique side AC first; and then the point E required for attaining such composition is fixed on the inert gas reduction line FB; then an intersecting point D of the combustible gas addition line connecting the apex C and the point E and the oblique side AB is fixed; so that the composition corresponding to the point D becomes a composition of the [combustion assisting gas and inert gas]-containing gas required for attaining the proportions of the combustion assisting gas and the combustible gas contained in the [combustion assisting gas, inert gas, and combustible gas]-containing gas to be produced.

It is possible to produce an [oxygen, water vapor, olefin, and hydrogen]-containing gas which comprises water vapor, oxygen, an olefin, and hydrogen by the second combustible gas mixing step (3) wherein hydrogen as the second combustible gas is mixed with the [oxygen, water vapor, and olefin]-containing gas (corresponding to the first mixed gas) that is obtained by reducing an amount of water vapor as described above, contains an olefin (e.g. propylene) as the first combustible gas, and is reduced in the amount of water vapor.

In the case of producing the [oxygen, water vapor, olefin, and hydrogen]-containing gas as described above, a composition of the [oxygen, water vapor, olefin, and hydrogen]-containing gas which is obtained by the hydrogen mixing step is preferably outside the explosion range of an oxygen-olefin-hydrogen system.

In one particularly preferred embodiment, an amount of hydrogen to be mixed in the second mixing step (3) with the [oxygen, water vapor, and olefin]-containing gas with the reduced amount of water vapor is selected in such a manner that the hydrogen addition line connecting the point corresponding to an olefin concentration (provided that only the amounts of oxygen and the olefin are considered, and amounts of the other component(s) is not considered in the case where the other component(s) is present in addition to oxygen and the olefin) in the [oxygen, water vapor, and olefin]-containing gas with the reduced amount of water vapor and an apex of 100% of oxygen, 0% of olefin, and 0% of hydrogen does not intersect with the explosion range of the oxygen-olefin-hydrogen system.

In other words using a broader concept, it is possible to produce a [combustion assisting gas, inert gas, first combustible gas, and second combustible gas]-containing gas (corresponding to the second mixed gas) by the second combustible mixing step (3) wherein the second combustible gas as another combustible gas is mixed with the [combustion assisting gas, inert gas and first combustible gas]-containing gas (corresponding to the first mixed gas) that is obtained by reducing an amount of the inert gas as described above, contains the first combustible gas as the combustible gas, and is reduced in the amount of the inert gas.

In the case of producing the second mixed gas as described above, the composition of the [combustion assisting gas, inert gas, first combustible gas, and second combustible gas]-containing gas obtained by the second combustible gas mixing step (provided that only the amounts of the combustion assisting gas, the first combustible gas, and the second combustible gas are considered, and the amounts of the inert gas and the other component(s) (if any) are not considered) is preferably outside the explosion range of the combustion assisting gas-first combustible gas-second

combustible gas system.

In a particularly preferred embodiment, an amount of the second combustible gas to be mixed with the [combustion assist gas, inert gas and first combustible gas]-containing gas with the reduced amount of the inert gas in the second mixing step (3) is selected in such a manner that the second combustible gas addition line connecting a point corresponding to the first combustible gas concentration in the [combustion assisting gas, inert gas and first combustible gas]-containing gas with the reduced amount of the inert gas (provided that only the amounts of the combustion assisting gas and the first combustible gas are considered, and other component(s) is not considered in the case where the other component(s) is present in addition to the combustion assisting gas and the first combustible gas) and an apex of 0% of the combustion assisting gas, 0% of the first combustible gas, 100% of the second combustible gas does not intersect with the explosion range of the combustion assisting gas-first combustible gas-second combustible gas system in the equilateral triangle coordinate graph indicating such explosion range

The equilateral triangle coordinate graphs indicating the explosion range of the combustion assisting gas-first combustible gas-second combustible gas system is schematically shown in Fig. 5. As to such a graph, the explosion range of the three-component system can be obtained according to the literatures or experimentally. Alternatively, the explosion range of Fig. 5 may be obtained by employing Le Chatelier's law. The apex A and the apex C are the same as those of the graph of Fig. 4, and the apex J corresponds to a composition wherein an amount of the second combustible gas (e.g. hydrogen) is 100%. A range enclosed by the oblique sides AC and AJ of the equilateral triangle, a curve LM, and a curve KN and indicated with shading is the explosion range.

The composition of the [combustion assisting gas, inert gas, and combustible gas]-containing gas with the reduced amount of the inert gas that is obtained by the method for producing the first mixed gas of the present invention is indicated by the point F as in Fig. 4. In the method for producing the second mixed gas of the present invention, when the second combustible gas is gradually added to and mixed with the [combustion assisting gas, inert gas, and combustible gas]-containing gas with the reduced amount of the inert gas, a composition of the gas after the mixing changes from the point F to the apex J. Therefore, the second combustible gas is added so as to arrive at a point P at which the [combustion assisting gas, first combustible gas, and second combustible gas]-containing gas which comprises predetermined amounts of the inert gas, the combustion assisting gas, the first combustible gas, and the second combustible gas is obtained. As is easily understood, the point P is preferably not present inside the explosion range. Further, in the course of adding and mixing of the second combustible gas, the gas composition after the mixing more preferably does not get into the explosion range. That is, it is more preferable that the second gas addition line does not intersect with the explosion range.

Since the upper limit of explosion limit of propylene in oxygen is 53 vol% (see Literature 2; New Safety Engineering Handbook (Shin-anzen-kougaku Binran (in Japanese); edited by Safety Engineering Association (Anzen-kougaku Kyoukai in Japanese); Colona Publishing Company; July 20, 1999; Newest Version; p. 134 and Table 3.29), in the case where the inert gas is water vapor, the combustion assisting gas is oxygen, and the first combustible gas is propylene in the present invention, it is preferable to adjust the composition (an amount ratio of water vapor and oxygen) and the amount of the [oxygen and water vapor]-containing gas and the amount of the olefin to be mixed with the [oxygen and water vapor]-containing gas in the first mixing step (or in the first mixing apparatus) when mixing propylene with the [oxygen and water vapor]-containing gas in such a manner that the proportion of propylene with respect to oxygen and propylene after the mixing is more than 53 vol%, preferably within the range of 60 to 90 vol%, for example 65 vol%.

It is noted that it is possible to estimate the explosion range by combining the features that experimentally and experientially confirmed with literature values as described below:

> The explosion range of the propylene-oxygen-hydrogen system will be estimated. By assuming that the upper limit concentration and the lower limit concentration of the explosion of the mixed gas are in accordance with the Le Chatelier's expression (or equation), the upper and lower limit concentrations of the mixed gas are represented by points on straight lines connecting the upper limit concentrations and the lower limit concentrations respectively of propylene and hydrogen (Literature 3: Separate Volute of Chemical Industry (Bessatsu Kagaku Kougyou in Japanese)- Disaster Prevention Engineering (Bousai-kougaku in Japanese)- Problems of Pollution Countermeasure (Kougai Taisaku no Shomondai in Japanese) ; Kabushiki Kaisha Kagaku Kogyo-Sha; p. 183 (1966. 6. 15)). The upper and lower limit concentrations are obtainable from Literature 2, for example. Results of actual calculation using the upper and lower limit concentrations based on the Le Chatelier's law are shown as an equilateral triangle coordinate graph in Fig. 7. In Fig. 7, limit concentrations of the mixed gas based on Literature 3 are expressed by the straight lines connecting each limit concentrations of pure components, the area which is sandwiched by the straight lines is the explosion range.

The Le Chatelier's expression (see Literature 1) is as described below:

$$L = 100/(n_1/L_1 + n_2/L_2 + \ldots n_i/L_i \ldots)$$

wherein L represents a limit concentration of a combustible gas mixture; Li represents a limit concentration (vol%) of a component i; and ni represents a concentration [vol%] of the component i.

In a particularly preferred embodiment, the composition (the amount ratio between water vapor and oxygen) and the amount of the [oxygen and water vapor]-containing gas and the amount of the olefin to be mixed with the [oxygen and water vapor]-containing gas are adjusted in the first mixing step (or in the first mixing apparatus) in such a manner which satisfies the above mentioned limit oxygen concentration of less than 11.5 vol% and the above mentioned propylene proportion of larger than 53 vol% based on oxygen and propylene.

It is noted that as a result of the investigation of the inventor, calculation results (estimation of explosion limit of a mixed gas using Le Chatelier's expression) has been found in that, in the case where the proportion of propylene after the mixing with respect to oxygen and propylene is more than 53 vol% as described above, a concentration of a total amount of propylene and hydrogen is more than the upper limit of the explosion limit of the [oxygen, propylene, and hydrogen]-containing gas irrespective of an amount of hydrogen added to the [oxygen and propylene]-containing gas.

Therefore, in the present invention, when preparing the [oxygen, water vapor, and olefin]-containing gas by condensing and removing water vapor in the condensing step (or in the condenser), it is preferable to adjust the amount of the olefin with respect to the total amount of oxygen and the olefin in the [oxygen, water vapor, and olefin]-containing gas so as to exceed the explosion range. In the case where the olefin is propylene, with the above-described procedure, the amount of hydrogen and propylene, i.e. the amount of combustible gases, exceeds the explosion upper limit of the mixed gas containing hydrogen, propylene, and oxygen irrespective of the amount of hydrogen to be added after the procedure. Consequently, possibility of the explosion of such mixed gas is largely reduced.

As described above, the [oxygen and water vapor]-containing gas is prepared in the preliminary mixing step (or in the preliminary mixing apparatus) by mixing the oxygen-containing gas and water vapor. The oxygen-containing gas may be a pure oxygen gas or a gas containing impurities in addition to oxygen, such as air containing nitrogen and oxygen. As to the mixed gas obtained by mixing the oxygen-containing gas which contains such an impurity with water vapor and then mixing with the olefin, the limit oxygen concentration described above and also the explosion limit in oxygen described above are calculated based only on the amount of oxygen contained in the oxygen-containing gas, and it is possible to judge whether or not the oxygen concentration in the mixed gas is lower than the limit oxygen concentration and whether or not a composition of the mixed gas is outside the range of the explosion limit without causing any problems in practice. Various oxygen gases available for the industrial uses contain impurities in addition to oxygen depending on the production methods of the gases, but the above-described idea is applicable to such oxygen gas similarly to air.

For instance, an oxygen gas produced by the PSA method (pressure swing adsorption method) and an oxygen gas produced by the cryogenic separation contain about 10% or less of impurities, and it is possible to use such an oxygen gas in the present invention in the same manner as described above.

As is easily understood from the above descriptions, based on limit oxygen concentrations and explosion limits of various olefins obtainable from the literatures and/or experiments, it is possible to appropriately select the composition and the amount of the [oxygen and water vapor]-containing gas and the amount of the olefin to be added in the case of preparing an [oxygen, water vapor, and olefin]-containing gas and an [oxygen, water vapor, olefin, and hydrogen]-containing gas wherein the olefin is other than propylene.

The above-described methods and apparatuses for producing the mixed gases are particularly effective for using an olefin epoxidation reaction in combination. Specifically, the method and apparatus for producing the mixed gas of the present invention are particularly effective when used in combination with an olefin epoxidizing apparatus of an olefin or a method for producing, from an olefin, an epoxy compound corresponding to the olefin. Particularly preferred olefin epoxidation reactions to be used in combination are as described below.

The preferable epoxidation reaction is a reaction producing an epoxy compound which comprises reacting an olefin, oxygen and hydrogen in a liquid phase in the presence of at lease one titanosilicate selected from the group consisting of a crystalline titanosilicate having MEL structure, MTW structure, BEA structure, MWW structure or DON structure, a mesoporous titanosilicate and a lamellar titanosilicate, a noble metal catalyst, and a quinoid compound or a dihydro-form of a quinoid compound.

According to the preferable epoxidation reaction, the epoxy compound is effectively produced from the olefin, oxygen and hydrogen by using the quinoid compound or the like.

Typical examples of the noble metal catalyst used in the preferable epoxidation reaction typically include palladium, platinum, ruthenium, rhodium, iridium, osmium, gold, or an alloy or a mixture thereof. Preferably, the noble metal catalyst is palladium, platinum or gold. More preferred noble metal catalyst is palladium. Palladium can be used by adding thereto and mixing therewith a metal such as platinum, gold, rhodium, iridium or osmium. A preferred metal

to be added is platinum.

Further, these noble metal catalysts may also be added as noble metal compounds such as oxides or hydroxides of the noble metals. Alternatively, a noble metal compound can be filled as it is in a reactor, followed by reducing it partially or completely with hydrogen contained in starting materials of the reaction under reaction conditions.

Usually, a noble metal catalyst1 is used while supported on a carrier. The noble metal catalyst can also be used while supported on titanosilicate. The noble metal catalyst can also be used while supported on a carrier other than titanosilicate such as oxides, for example, silica, alumina, titania, zirconia, niobia, etc.; hydrates, for example, niobic acid, zirconic acid, tungstic acid, titanic acid, etc.; or carbon; and a mixture thereof. When the noble metal catalyst is supported on a carrier other than titanosilicate, the carrier supporting the noble metal catalyst can be mixed with titanosilicate and the mixture can be used as a catalyst. Among the supports other than titanosilicate, a preferred carrier is carbon. As known carbon carriers, there are active carbon, carbon black, graphite, carbon nano-tube and the like.

As a method for preparing the noble metal catalyst supported on the carrier, a colloidal solution of a noble metal obtained by dispersing particles of the noble metal with a dispersant such as citric acid, polyvinyl alcohol, polyvinyl pyrrolidone, sodium hexametaphosphate, etc. is supported on the carrier by an impregnation method or the like and calcined in an atmosphere of an inert gas. Alternatively, the noble metal catalyst supported on the carrier can also be prepared by having a noble metal compound which can be used as a noble metal source such as a nitrate salt of the noble metal, e.g., palladium nitrate, a sulfate salt of the noble metal, e.g., palladium sulfate dihydrate, a halogenide of the noble, e.g., palladium chloride, a carboxylate salt, e.g., palladium acetate or an ammine complex, e.g., Pd tetraammine chloride, supported on the carrier by an impregnation method or the like, followed by reduction with a reducing agent; or it can also be prepared by once changing the noble metal compound to its hydroxide with an alkali such as sodium hydroxide, followed by reduction with a reducing agent in a liquid phase or a gas phase. Examples of the reducing agent to be used in case of the reduction in the liquid phase include hydrogen, hydrazine monohydrate, formaldehyde, sodium borohydride, etc. When using hydrazine monohydrate or formaldehyde, the addition of an alkali is also known. Examples of the reducing agent to be used in case of the reduction in the gas phase include hydrogen, ammonia, and the like. The catalyst can also be prepared by calcining and reducing a carrier on which a noble metal source is supported in the presence of hydrogen gas. A preferred reduction temperature is varied depending on the noble metal source supported, but generally from 0°C to 500°C. Moreover, the catalyst can also be prepared by having an ammine complex of the noble metal, e.g., Pd tetraammine chloride supported on the carrier by an impregnation method or the like, followed by reduction with ammonia gas generated upon thermal decomposition in an atmosphere of an inert gas. The reduction temperature is varied depending on the ammine complex of the noble metal, but in case of using Pd tetraammine chloride, generally from 100°C to 500°C and preferably 200°C to 350°C.

In any of the methods, if necessary, it is possible to activate the resultant catalyst by a thermal treatment in an atmosphere of an inert gas, ammonia gas, vacuum, hydrogen or air. Further, after filling a compound of the noble metal such as an oxide or hydroxide thereof into a reactor, it can be reduced under reaction conditions.

In this way, the resultant carrier supporting the noble metal catalyst generally contains the noble metal catalyst in a range of 0.01 to 20% by weight, preferably 0.1 to 5% by weight.

The weight ratio of the noble metal catalyst to titanosilicate (weight of a noble metal / weight of titanosilicate) is preferably 0.01 to 100% by weight, more preferably 0.1 to 20% by weight.

Titanosilicate is a generic name of a substance in which a part of Si in a porous silicate ($SiO_2$) is replaced with Ti. Ti of titanosilicate is placed in an $SiO_2$ framework, and this can be easily confirmed by a peak of 210 to 230 mm in ultraviolet-visible absorption spectra.

In addition, Ti of $TiO_2$ is usually 6-coordination, whereas Ti of titanosilicate is 4-coordination. This can be easily confirmed by measuring coordination number in a Ti-K-edge XAFS analysis.

Examples of the titanosilicates used in the preferable epoxidation reaction includes crystalline titanosilicates such as, in terms of the framework type code by IZA (International Zeolite Association), TS-2 having MEL structure, Ti-ZSM-12 having MTW structure (e.g., one described in Zeolites 15, 236-242, (1995)), Ti-Beta having BEA structure (e.g., one described in Journal of Catalysis 199, 41-47, (2001)), Ti-MWW having MWW structure (e.g., one described in Chemistry Letters 774-775, (2000)), Ti-UTD-1 having DON structure (e.g., one described in Zeolites 15, 519-525, (1995)), TS-1 having MFI structure (e.g., one described in Journal of Catalysis, 130, (1991)), etc. Examples of the lamellar titanosilicate include a titanosilicate having a structure with expanded interlayers in MWW structure such as Ti-MWW precursor (e.g., one described in JP 2003-327425 A), Ti-YNU-1 (e.g. one described in Angewandte Chemie International Edition 43, 236-240, (2004)), etc. Mesoporous titanosilicate is a generic name of titanosilicates usually having periodic pore structures of diameters ranging from 2 to 10 nm and examples thereof include Ti-MCM-41 (e.g., one described in Microporous Materials 10, 259-271, (1997)), Ti-MCM-48 (e.g., one described in Chemical Communications 145-146, (1996)), Ti-SBA-15 (e.g., one described in Chemistry of Materials 14, 1657-1664, (2002)), etc. Further examples of the titanosilicate include a titanosilicate having features of both mesoporous titanosilicate and titanosilicate zeolite, such as Ti-MMM-1 (e.g. one described in Microporous and Mesoporous Materials 52, 11-18, (2002)).

Among the titanosilicates used in the preferable epoxidation reaction, a crystalline titanosilicate or a lamellar

titanosilicate which has pores of 12 or more membered oxygen rings is preferred. As the crystalline titanosilicate having pores of 12 or more membered oxygen rings, Ti-ZSM-12, Ti-Beta, Ti-MWW and Ti-UTD-1 are mentioned.

As the lamellar titanosilicate having pores of 12 or more membered oxygen rings, Ti-MWW precursor and Ti-YNU-1 are mentioned. As a more preferred titanosilicate, Ti-MWW and Ti-MWW precursor are mentioned.

Usually, the titanosilicate used in the preferable epoxidation reaction can be synthesized by such a method that a surfactant is used as a template or a structure directing agent, a titanium compound and a silicon compound are hydrolyzed, if necessary, followed by improvement of crystallization or periodic regularity of pores by hydrothermal synthesis etc., and then the surfactant is removed by calcining or extraction. Usually, the crystalline titanosilicate having MWW structure is prepared as follows. Namely, a silicon compound and a titanium compound are hydrolyzed in the presence of a structure directing agent to prepare a gel. Then, the resultant gel is subjected to heat treatment in the presence of water such as hydrothermal synthesis, etc. to prepare a lamellar precursor of crystal. Then, the resultant lamellar precursor of crystal is subjected to crystallization by calcination to prepare the crystalline titanosilicate having MWW structure.

The titanosilicate used in the preferable epoxidation reaction includes one silylized with a silylizing agent such as 1,1,1,3,3,3-hexamethyldisilazan, etc. Since silylization further enhances activity or selectivity, a silylized titanosilicate is also a preferred titanosilicate (for example, silylized Ti-MWW, etc.).

In addition, the titanosilicate can be used after it is activated by treatment with a hydrogen peroxide solution at an appropriate concentration. Usually, the concentration of the hydrogen peroxide solution can be in a range of 0.0001% to 50% by weight. The solvent of hydrogen peroxide solution is not particularly limited, but water or a solvent used for a propylene oxide synthesis reaction is convenient and preferable from the industrial view point.

As the quinoid compound, there are two types, i.e., a p-quinoid compound and an o-quinoid compound, both are included in the quinoid compound used in the present invention.

Examples of the quinoid compound include a phenanthraquinone compound and a p-quinoid compound represented by the formula (1):

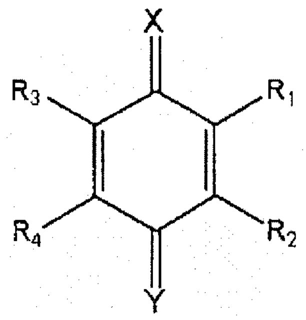

(1)

wherein $R_1$, $R_2$, $R_3$ and $R_4$ represent a hydrogen atom or, each adjacent $R_1$ and $R_2$ , or $R_3$ and $R_4$ are independently bonded at their terminal ends, and form a benzene ring optionally substituted with an alkyl group or a hydroxyl group, or a naphthalene ring optionally substituted with an alkyl group or a hydroxyl group together with the carbon atoms of the quinone to which they are bonded, and X and Y are the same or different from each other and represent an oxygen atom or a NH group.

Examples of the compound represented by the formula (1) include

(1) a quinone compound (1A): the compound represented by the formula (1), wherein $R_1$, $R_2$, $R_3$ and $R_4$ are a hydrogen atom, and both X and Y are an oxygen atom;

(2) a quinone-imine compound (1B): the compound represented by the formula (1), wherein $R_1$, $R_2$, $R_3$ and $R_4$ are a hydrogen atom, X is an oxygen atom, and Y is a NH group; and

(3) a quinone-diimine compound (1C): the compound represented by the formula (1), wherein $R_1$, $R_2$, $R_3$ and $R_4$ are a hydrogen atom, and both X and Y are a NH group.

The quinoid compound of the formula (1) includes an anthraquinone compound represented by the formula (2):

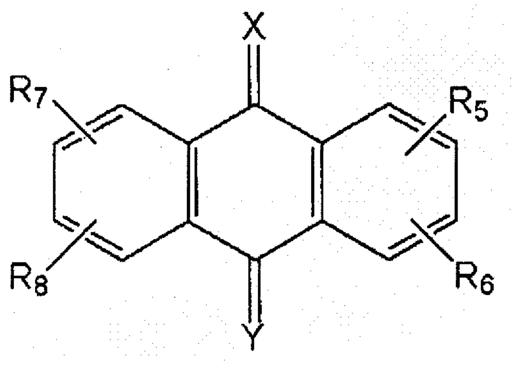

(2)

wherein X and Y are as defined in the formula (1), $R_5$, $R_6$, $R_7$ and $R_8$ are the same or different from each other, and represent a hydrogen atom, a hydroxyl group, or an alkyl group (e.g., $C_1$-$C_5$ alkyl such as methyl, ethyl, propyl, butyl, pentyl, etc.).

In the formula (1) and formula (2), preferably, X and Y represent an oxygen atom. The quinoid compound represented by the formula (1) wherein X and Y are an oxygen atom is particularly referred to as a quinone compound or a p-quinone compound, and the quinoid compound represented by the formula (2) wherein X and Y are an oxygen atom is particularly referred to as anthraquinone compound.

Examples of the dihydro-form of the quinoid compound include dihydro-forms of the compounds represented by the foregoing formulas (1) and (2), i.e. compounds represented by the formulas (3) and (4):

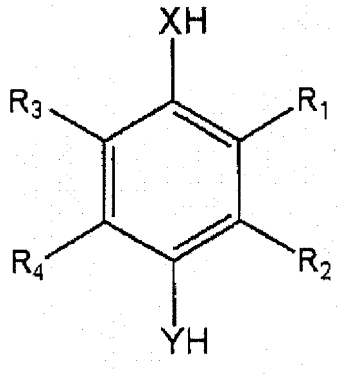

(3)

wherein $R_1$, $R_2$, $R_3$, $R_4$, X and Y are as defined in the foregoing formula (1); and

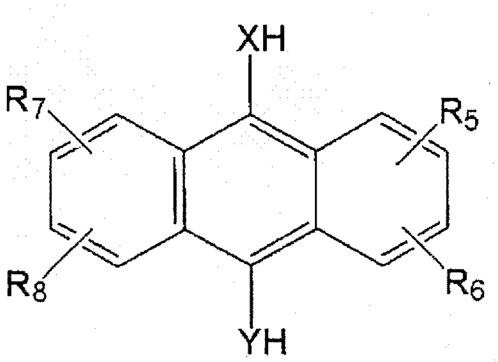

(4)

wherein X, Y, $R_5$, $R_6$, $R_7$ and $R_8$ are as defined in the foregoing formula (2).

In the formula (3) and formula (4), preferably, X and Y represent an oxygen atom. The dihydro-form of the quinoid compound represented by the formula (3) wherein X and Y are an oxygen atom is particularly referred to as a dihydroquinone compound or a dihydro p-quinone compound, and the dihydro-form of the quinoid compound represented by the formula (4) wherein X and Y are an oxygen atom is particularly referred to as a dihydroanthraquinone compound.

Examples of the phenanthraquinone compound include 1,4-phenanthraquinone as the p-quinoid compound and 1,2-, 3,4-, and 9,10-phenanthraquinones as the o-quinoid compounds.

Specific examples of the quinone compound include benzoquinone, naphthoquinone, anthraquinone, 2-alkylanthraquinone compounds such as 2-ethylanthraquinone, 2-t-bytylanthraquinone, 2-amylanthraquinone, 2-methylanthraquinone, 2-butylanthraquinone, 2-t-amylanthraquinone, 2-isopropylanthraquinone, 2-s-butylanthraquinone and 2-s-amylanthraquinone, 2-hydroxyanthraquinone, polyalkylanthraquinone compounds such as 1,3-diethylanthraquinone, 2,3-dimethylanthraquinone, 1,4-dimethylanthraquinone and 2,7-dimethylanthra.quinone, poylhydroxyanthraquinone such as 2,6-dihydroxyanthraquinone, naphthoquinone and a mixture thereof.

Preferred examples of the quinoid compound include anthraquinone, and 2-alkylanthraquinone compounds (in formula (2), X and Y are an oxygen atom, $R_5$ is an alkyl group substituted at 2 position, $R_6$ represents a hydrogen atom, and $R_7$ and $R_8$ represent a hydrogen atom). Preferred examples of the dihydro-form of the quinoid compound include dihydro-forms that corresponds to these preferred quinoid compounds.

The addition of the quinoid compound or the dihydro-form of the quinoid compound (hereinafter, abbreviated as the quinoid compound derivative) to a reaction solvent can be carried out by first dissolving the quinoid compound derivative in a liquid phase and then subjecting it to the reaction. For example, a hydride compound of the quinoid compound such as hydroquinone or 9,10-anthracenediol may be added to a liquid phase, followed by oxidation with oxygen in a reactor to generate the quinoid compound and use it in the reaction.

Further, the quinoid compounds used in the present invention including the quinoid compounds exemplified above may become dihydro-forms of partly hydrogenated quinoid compounds depending on reaction conditions, and these compounds may also be used.

Hereinafter, the amount of the quinoid compound to be used will be explained. In the present invention, the amount of the dihydro-form of the quinoid compound can be set similarly to that of the quinoid compound.

Usually, the amount of the quinoid compound to be used can be in a range of 0.001 mmol/kg to 500 mmol/kg per unit weight of a solvent (unit weight of water, an organic solvent or a mixture thereof). A preferred amount of the quinoid compound is 0.01 mmol/kg to 50 mmol/kg.

Usually, the preferable epoxidation reaction is carried out in a liquid phase of water, an organic solvent or a mixture thereof. To the liquid phase is added the quinoid compound, the dihydro-form of quinoid compound or a mixture thereof, and the quinoid compound derivative is preferably used by dissolving it in the liquid phase.

Since the quinoid compound derivative tends to act more efficiently when the liquid phase contains an organic solvent, an epoxy compound can be obtained with a good selectivity even when an amount of the quinoid compound derivative used is reduced as compared with the case where the liquid phase contains no organic solvent. The organic solvent of the present invention means an organic compound which alone is liquid at a reaction temperature and a reaction pressure. Examples of the organic solvent include alcohols, ketones, nitriles, ethers, aliphatic hydrocarbons, aromatic hydrocarbons, halogenated hydrocarbons, esters, glycols, or a mixture thereof. As the alcohol, methanol, 2-methyl-2-propanol may be exemplified. Examples of the suitable solvent which can suppress sequentially production of by-products due to a reaction with water or alcohol in a synthesis reaction of an epoxy compound include linear or branched saturated aliphatic nitriles and aromatic nitriles. Examples of these nitrile compounds include $C_2$-$C_4$ alkyl nitrile such as acetonitrile, propionitrile, isobutyronitrile and butyronitrile, and benzonitrile, with acetonitrile being preferred.

In the case where a mixture of water and an organic solvent is used, usually, the ratio of water and the organic solvent is 90 : 10 to 0.01 : 99.99 by weight, preferably 50 : 50 to 0.01 : 99.99. When the ratio of water is too large, sometimes, an epoxy compound is apt to react with water, which causes deterioration due to ring opening, resulting in lowering the selectivity of the epoxy compound. To the contrary, when the ratio of an organic solvent is too large, recovery costs of the solvent becomes high.

In the process of the present invention, it is also effective to add a salt selected from an ammonium salt, an alkyl ammonium salt or an alkyl aryl ammonium salt to the reaction solvent together with the titanosilicate, the noble metal catalyst and the quinoid compound, because such a salt can prevent the lowering of catalyst activity or can further increase catalyst activity to enhance utilization efficiency of hydrogen. Usually, the amount of the salt selected from the ammonium salt, the alkyl ammonium salt or the alkyl aryl ammonium salt to be added is 0.001 mmol/kg to 100 mmol/kg per unit weight of the solvent (in the case of a mixture of water and an organic solvent, the total weight thereof).

Examples of the salt selected from the ammonium salt, the alkyl ammonium salt or the alkyl aryl ammonium salt include a salt composed of:

(1) an anion selected from, a sulfate ion, a hydrogen sulfate ion, a carbonate ion, a hydrogen carbonate ion, a phosphate ion, a hydrogen phosphate ion, a dihydrogen phosphate ion, a hydrogen pyrophosphate ion, a pyrophosphate ion, a

halogen ion, a nitrate ion, a hydroxide ion or a $C_1$-$C_{10}$ carboxylate ion; and (2) a cation selected from ammonium, an alkyl ammonium or an alkyl aryl ammonium.

Examples of the $C_1$-$C_{10}$ carboxylate ion include formate ion, acetate ion, propionate ion, butyrate ion, valerate ion, caproate ion, caprylate ion and caprinate ion.

Examples of the alkyl ammonium include tetramethylammonium, tetraethylammonium, tetra-n-propylammonium, tetra-n-butylammonium and cetyltrimethylammonium.

Preferred examples of the salt composed of the ammonium, the alkyl ammonium or the alkyl aryl ammonium include ammonium salts of inorganic acids such as ammonium sulfate, ammonium hydrogen sulfate, ammonium carbonate, ammonium hydrogen carbonate, diammonium hydrogen phosphate, ammonium dihydrogen phosphate, ammonium phosphate, ammonium hydrogen pyrophosphate, ammonium pyrophosphate, ammonium chloride and ammonium nitrate; or ammonium salts of $C_1$ to $C_{10}$ carboxylic acids such as ammonium acetate, and a preferred ammonium salt is ammonium dihyrogen phosphate.

The olefin used in the preferable epoxidation reaction means a hydrocarbon having one or more carbon-carbon double bonds. Examples of the olefin used in the present invention include aliphatic olefins such as ethylene, propylene, 1-butene, 2-butene, 1-pentene, 1-hexene and 2-hexene; cyclic olefins such as cyclopentene and cyclohexene; diolefins such as butadiene; and an olefin having an aromatic ring such as styrene, etc.

The preferable epoxidation reaction can be preferably used in the production of the epoxy compounds such as ethylene oxide, propylene oxide, 1-butene oxide, 2-butene oxide, 1-pentene oxide, 1-hexene oxide and 2-hexene oxide from corresponding $C_2$-$C_6$ olefins such as ethylene, propylene, 1-butene, 2-butene, 1-pentene, 1-hexene, 2-hexene, cyclopentene, cyclohexene and butadiene.

Examples of oxygen to be used for the preferred epoxidation reaction include an oxygen gas, air, and the like. As the oxygen gas, inexpensive one obtained by the pressure swing method is usable, and a high purity oxygen gas produced by the cryogenic separation may be used as required. As to hydrogen to be used, there is no limitation for the production of hydrogen. For example, those produced by steam reforming, partial oxidation, catalytic oxidation or electrolysis of a fossil material such as hydrocarbons, or electrolysis, thermochemical decomposition, biomass conversion or photodecomposition of nonfossile materials.

In performing the preferred epoxidation reaction as described above, oxygen, the olefin, and hydrogen are supplied to the rector. The above-described method or apparatus for producing the mixed gas of the present invention is applicable to the supply. Specifically, the mixed gas is obtained by performing

(1) the first mixing step for preparing the [oxygen, water vapor, and olefin]-containing gas which comprises oxygen, water vapor, and the olefin by mixing the [oxygen and water vapor]-containing gas which comprises oxygen and water vapor with the olefin;
(2) the condensation step for preparing the [oxygen, water vapor, and olefin]-containing gas of which amount of water vapor is reduced by condensing water vapor contained in the [oxygen, water vapor, and olefin]-containing gas followed by removing water obtained by condensation; and
(3) the second mixing step for mixing, with hydrogen, the [oxygen, water vapor, and olefin]-containing gas with the reduced amount of water vapor to prepare the [oxygen, water vapor, olefin, and hydrogen]-containing gas which comprises oxygen, water vapor, the olefin, and hydrogen. Thus prepared mixed gas is supplied to the reactor performing the epoxidation reaction.

It is noted that water vapor present in the mixed gas does not exert substantially adverse influence on the epoxidation reaction since the amount thereof is small. Particularly, since the reaction system includes water in the case of using a liquid containing water as the reaction solvent, water vapor that is present in the mixed gas does not exert substantially adverse influence on the epoxidation reaction.

The reactor to be used is not particularly limited, and any appropriate reactor may be used. Examples of a procedure for the preferred epoxidation reaction include a fixed bed reaction, a stirred tank type reaction, a fluid bed reaction, a moving bed reaction, a bubble column reaction, a tubular reaction, a circulation reaction, and the like. As a particularly preferred reactor, those provided with a function of circulating a vapor phase or a liquid phase of the reactor, and examples of such a reactor include those described below:

- Reactors provided with a function of externally circulating a vapor phase by a compressor, wherein the mixed gas to be supplied (i.e. the [oxygen, water vapor, olefin, and hydrogen]-containing gas) is supplied to the reactor while being added to the externally circulated vapor phase;
- Reactors provided with a function of externally circulating a liquid phase of the reactor via an ejector,

wherein the mixed gas to be provided is supplied to the reactor while being sucked by the ejector (if necessary, a part

of the vapor phase of the reactor may be sucked together with the mixed gas by the ejector);

- Reactors provided with a function of forced internally circulating a vapor phase to a liquid phase, particularly, a reactor provided with a so-called self-sucking type gas-liquid contact stirrer; and
- Reactors having a stirrer capable of entraining a part of a vapor phase on a liquid phase into the liquid phase.

A partial pressure ratio between oxygen and hydrogen to be supplied to the reactor in the preferred epoxidation reaction may be usually within the range of 1:50 to 50:1. A preferred partial pressure ratio between oxygen and hydrogen is 1:2 to 10:1. When the oxygen/hydrogen pressure ratio is too high, a production rate of the epoxy compound may be reduced. In addition, when the oxygen/hydrogen partial pressure ratio is too low, the selectivity of the epoxy compound may be reduced due to an increase in paraffin by-products.

A reaction temperature in the preferred epoxidation reaction is usually 0°C to 150°C, preferably 40°C to 90°C. The reaction speed is reduced when the reaction temperature is too low, and the by-products are increased due to a side reaction when the reaction temperature is too high.

In a preferred epoxidation reaction, a reaction pressure is not particularly limited, and it is usually 0.1 to 20 MPa, preferably 1 to 10 MPa, in a gauge pressure.

The reaction speed is reduced due to insufficient dissolution of raw material gases when the reaction pressure is too low. The cost for devices relating to the reaction is increased when the reaction pressure is too high. Recovery of the epoxy compound that is the product of the present invention is performed by the conventional distillation separation, and non-reacted olefin and solvents may be separated by distillation, membrane filtration, or the like as required.

It is possible to suitably combine the method or apparatus for producing the mixed gas of the present invention with the epoxidation reaction as described above. Schematically shown in Fig. 1 is a flow sheet of a process for producing an epoxy compound of propylene (i.e. propylene oxide) by using the epoxidation reaction in combination.

In Fig. 1,

the preliminary mixing step is performed for preparing an [oxygen and water vapor]-containing gas by mixing oxygen and water vapor in a preliminary mixing apparatus (e.g. static mixer) 10;

then, the first mixing step is performed for preparing an [oxygen, water vapor, and propylene]-containing gas which comprises oxygen, water vapor, and propylene by supplying thus obtained [oxygen and water vapor]-containing gas to a first mixing apparatus (e.g. static mixer) 12, and also by supplying propylene to the first mixing apparatus so as to mix these gases; and

then the condensation step is performed for obtaining an [oxygen, water vapor, and propylene]-containing gas with the reduced amount of water vapor by supplying the [oxygen, water vapor, and propylene]-containing gas to a condenser 14 so as to condense a part of, preferably most of, water vapor (preferably so as to condense in such a manner that water vapor corresponding to a saturated water vapor pressure under the operation conditions substantially remains as a gas), and discharging the condensed water as a drain 24 simultaneously with obtaining the [oxygen, water vapor, and propylene]-containing gas.

Subsequently, the second mixing step is performed for preparing an [oxygen, water vapor, propylene, and hydrogen]-containing gas which comprises oxygen, water vapor, propylene, and hydrogen by supplying the [oxygen, water vapor, and propylene]-containing gas with the reduced amount of water vapor to a second mixing apparatus (e.g. static mixer) 16, and also by supplying hydrogen to the second mixing apparatus so as to mix these gases. The obtained [oxygen, water vapor, propylene, and hydrogen]-containing gas is added to an appropriate part of a line 22 which externally circulates around the reactor 18 a vapor phase part taken out from an epoxidizing reactor 18 and compressed by a compressor 20 , so that the [oxygen, water vapor, propylene, and hydrogen]-containing gas is supplied to a bottom part of the reactor 18 together with the compressed gas and thus to a liquid phase of the epoxidation reaction.

In Fig. 2, the preparation of the [oxygen, water vapor, and olefin]-containing gas with the reduced amount of water vapor and also the preparation of the [oxygen, water vapor, olefin, and hydrogen]-containing gas are the same as those of Fig. 1, but the reactor 18 is substantially different from that of Fig. 1 in the feature of having an ejector 26. The liquid phase taken out from the reactor is supplied to the ejector 26, and a negative pressure is caused by the ejector. The [oxygen, water vapor, olefin, and hydrogen]-containing gas supplied to the ejector is mixed with the liquid phase passing through the ejector by means of such negative pressure, and supplied to the reactor together with the liquid phase. It is noted that in the embodiment shown in the drawing, the line is so connected that a part of a vapor phase of the reactor is also sucked by the ejector together with the [oxygen, water vapor, olefin, and hydrogen]-containing gas.

Shown in Fig. 3 is a modification example of the embodiment shown in Fig. 2. In the process shown in Fig. 3, a gas-liquid mixture fluid generated by the condensation operation in the condenser 14 is supplied to a gas-liquid separator (e.g. a separation drum) 28, and the separated gas is supplied to the reactor 18 while supplying the liquid phase (drain water) is supplied to the condenser. The liquid phase may be supplied to the condenser 14 after being brought into a boiling state by flushing at a reduced pressure. By supplying the liquid phase to the condenser, it is

possible to effectively use a heat of the [oxygen, water vapor, and olefin]-containing gas with the reduced amount of water vapor.

It is noted in the embodiment shown in Fig. 3, the second mixing apparatus 16 for mixing the [oxygen, water vapor, and olefin]-containing gas of which amount of water vapor is reduced with hydrogen mixes together the vapor phase taken out from the reactor. In the embodiment of Fig. 3, the second mixing apparatus 16 is a connection part of the piping. Therefore, based on the embodiment of Fig. 3, the second mixing step (or the second mixing apparatus) of the present invention, the [oxygen, water vapor, olefin, and hydrogen]-containing gas which comprises oxygen, water vapor, the olefin, and hydrogen is prepared while mixing the vapor phase together upon mixing, with hydrogen, the [oxygen, water vapor, and olefin]-containing gas of which amount of water vapor is reduced. Therefore, the mixing of the [oxygen, water vapor, and olefin]-containing gas with hydrogen in the second mixing step (or in the second mixing apparatus) in the method or apparatus for producing the mixed gas of the present invention encompasses an embodiment in which such mixing is carried out in the presence of other gas (e.g. the vapor phase taken out from the reactor).

In any one of embodiments, at least one of (or all of, depending on the case) the preliminary mixing apparatus, the first mixing apparatus, and the second mixing apparatus may not be a special mixer and may be a part at which the pipes are connected, i.e. a position at which gases are joined. Therefore, in the present invention, the mixing apparatus may be a point where the fluids are joined, for example, a connection part of pipes in which different gases flow.

Further, in another embodiment, hydrogen may be supplied to the epoxidizing reactor separately from the supply of the [oxygen, water vapor, and olefin]-containing gas with the reduced amount of water vapor that is obtained by the condensation step to the epoxidizing reactor.

EXAMPLES

[Example 1]

An oxygen gas (flow rate: 16155 $Nm^3$/Hr; temperature: 200°C; pressure: 4 MPa) and water vapor (flow rate: 185787 $Nm^3$/Hr; temperature: 250°C; pressure: 4 MPa) are mixed by using a line mixer as the preliminary mixing apparatus so as to obtain an [oxygen and water vapor]-containing gas which contains oxygen and water vapor. The oxygen concentration in the [oxygen and water vapor]-containing gas is about 8 % by volume.

Further, the [oxygen and water vapor]-containing gas and propylene (flow rate: 30228 $Nm^3$/Hr; temperature: 200°C; pressure: 4 MPa) are mixed by using a line mixer as the first mixing apparatus so as to obtain an [oxygen, water vapor, and propylene]-containing gas which contains oxygen, water vapor, and propylene. In the [oxygen, water vapor, and propylene]-containing gas, propylene was about 65 % by volume.

Next, the [oxygen, water vapor, and propylene]-containing gas is passed through tubes of a vertical multi-tubular heat exchanger used as a first condenser to be cooled to about 125°C, followed by a condensing step for cooling to 100°C using a vertical multi-tubular heat exchanger used as a second condenser, thereby obtaining a gas-liquid mixture fluid. The gas-liquid mixture fluid is subjected to gas-liquid separation in a gas-liquid separation drum so as to obtain an [oxygen, water vapor, and propylene]-containing gas with the reduced amount of water vapor that contains 34 % by volume of oxygen, 3.7 % by volume of water vapor, and 62.3 % by volume of propylene as well as a condensed water of 149.5 tons/Hr.

The thus-obtained [oxygen, water vapor, and propylene]-containing gas with the reduced amount of water vapor is supplied to a reactor together with hydrogen to be used for an epoxidation reaction. The reactor having an ejector capable of attaining a high gas sucking rate is used as the reactor, and the reactor is so structured that the [oxygen, water vapor, and propylene]-containing gas with the reduced amount of water vapor is sucked by the ejector so that the gas is supplied to the reactor. With the reactor, the ejector self-sucks the gas of the reactor gas phase, and disperse the gas into a liquid phase of the reactor.

The piping for the obtained [oxygen, water vapor, and propylene]-containing gas with the reduced amount of water vapor is connected to a self-sucking piping of the vapor phase gas of the reactor which includes the ejector so as to mix, with the vapor phase gas of the reactor, the obtained [oxygen, water vapor, and propylene]-containing gas with the reduced amount of water vapor. Further, a piping is connected so as to supply hydrogen (18290 $Nm^3$/hr) to the thus-mixed gas, so that the mixed gases are supplied as an intake gas of the ejector, and also the gases are dispersed in the reaction liquid (corresponding to the embodiment shown in Fig. 3).

A circulation line for externally circulating the liquid phase via the ejector is provided, and the circulation line is provided with a heat exchanger (water is passed through its shell side) for removing a reaction heat. A mixture of Ti-MWW catalyst and 1% palladium carrying active carbon catalyst is provided inside the heat exchanger as a fixed bed.

To the liquid phase of the reactor, 219 tons/Hr of acetonitrile water (water/acetonitrile weight ratio is 30/70) containing 20 ppm of anthraquinone and 20 ppm of ammonium dihydrogenphosphate is continuously supplied. During the reaction, the reaction was performed while adjusting a reaction temperature to 50°C and a reaction pressure to 4 MPa. As a result of the reaction, 33 tons/Hr of a liquid containing propylene oxide is obtained.

The vapor phase of the reactor is so operated that a major part of the vapor phase is basically occupied by the inert gas component, but, in the case where an amount of the inert gas of the vapor phase is dissolved into the liquid to cause insufficient control of the reaction pressure, an inert gas such as nitrogen is supplied to the reactor while mixed with the gas to be supplied to the reactor.

By reducing a pressure to a pressure of 0.1 MPa, 3.7 tons/Hr of water vapor of 92.4°C and 145.7 tons/Hr of a boiling liquid is obtained from the condensed water obtained by the gas-liquid separation drum. The boiling liquid is passed through the shell side of the vertical multi-tubular heat exchanger used as the first condenser so as to use as a coolant for cooling the [oxygen, water vapor, and propylene]-containing gas which flows through the tubes of the vertical multi-tubular heat exchanger so that water vapor of a temperature of 151.7°C, a pressure of 0.1 MPa, and a flow rate of 145.7 tons/Hr is recovered by receiving heat from the inside of the tubes. The water vapor is compressed to 4 MPa by using a compressor, and further water vapor of which amount corresponds to that of the water vapor that has been contained in the [oxygen, water vapor, and propylene]-containing gas with the reduced amount of water vapor may be added to the compressed water vapor to obtain mixed water vapor, so that it is possible to reuse the mixed water vapor as water vapor for preparing the [oxygen and water vapor]-containing gas.

The present invention provides a safer means for preparing a mixed gas of a combustible gas (e.g. an olefin such as propylene and/or hydrogen) and oxygen, and such means is suitably used for epoxidizing an olefin such as propylene.

In addition, in the case of using propylene as the combustible gas, it is possible to use a [combustion assisting gas (e.g. oxygen), inert gas (e.g. water vapor), and propylene]-containing mixed gas that is more safely prepared with a reduced amount of inert gas for production of acrolein or the like.

In addition, as is easily understood from the foregoing descriptions, in preparing a mixture of the combustion assisting gas and the combustible gas, it is possible to prepare a mixture of the combustion assisting gas and the combustible gas more safely by mixing the combustible gas with the combustion assisting gas that is diluted with the inert gas. As is easily understood by those skilled in the art, it is possible to produce various mixtures of various combustible gases and various combustion assisting gases without departing from the sprit of the present invention.

For example, it is possible to use ethylene as the combustible gas, it is possible to more safely obtain a [combustion assisting gas (e.g. oxygen), inert gas (e.g. water vapor), and ethylene]-containing mixed gas with the reduced amount of the inert gas, and it is possible to use the mixed gas for the production of ethylene oxide, acetaldehyde, and the like.

For example, it is possible to use acrolein as the combustible gas, it is possible to more safely obtain a [combustion assisting gas (e.g. oxygen), inert gas (e.g. water vapor), and acrolein]-containing mixed gas with the reduced amount of the inert gas, and it is possible to use the mixed gas for the production of acrylic acid and the like.

For example, it is possible to use isobutene as the combustible gas, it is possible to more safely obtain a [combustion assisting gas (e.g. oxygen), inert gas (e.g. water vapor), and isobutene]-containing mixed gas with the reduced amount of the inert gas, and it is possible to use the mixed gas for the production of methacrolein and the like.

For example, it is possible to use methacrolein as the combustible gas, it is possible to more safely obtain a [combustion assisting gas (e.g. oxygen), inert gas (e.g. water vapor), and methacrolein]-containing mixed gas with the reduced amount of the inert gas, and it is possible to use the mixed gas for the production of methacrylic acid and the like.

Finally, the present application includes the following inventions:

Invention 1. A method for producing a mixed gas which comprises a combustion assisting gas and a combustible gas **characterized in that** the method comprises the steps of:

(1) mixing a [combustion assisting gas and inert gas]-containing gas which comprises the combustion assisting gas and an inert gas with the comestible gas so as to prepare a [combustion assisting gas, inert gas, and combustible gas]-containing gas which comprises the combustion assisting gas, the inert gas, and the combustible gas; and

(2) reducing an amount of the inert gas contained in the [combustion assisting gas, inert gas, and combustible gas]-containing gas.

Invention 2. The method according to invention 1 **characterized in that** the step (1) adjusts a composition and/or an amount of the [combustion assisting gas and inert gas]-containing gas as well as an amount of the combustible gas to be mixed and mixes these gases in such a manner that a composition of the [combustion assisting gas, inert gas, and combustible gas]-containing gas which is obtained after the mixing is positioned outside the explosion range of a combustion assisting gas-inert gas-combustible gas system.

Invention 3. The method according to invention 2 **characterized in that** a ratio between the combustion assisting gas and the inert gas contained in the [combustion assisting gas and inert gas]-containing gas to be mixed with the combustible gas in the step (1) is selected such that, in an equilateral triangle coordinate graph indicating the

explosion range of the combustion assisting gas-inert gas-combustible gas system, a combustible gas addition line connecting a point corresponding to a combustion assisting gas concentration in the [combustion assisting gas and inert gas]-containing gas (provided that only amounts of the combustion assisting gas and the inert gas are considered, and an amount(s) of the other component(s) is not considered when such other component(s) is present in addition to the combustion assisting gas and the inert gas) and an apex of 0% of the combustion assisting gas, 0% of the inert gas, and 100% of the combustible gas does not intersect the explosion range of the combustion assisting gas-inert gas-combustible gas system.

Invention 4. The method according to invention 3 **characterized in that** an amount of the combustible gas to be mixed with the [combustion assisting gas and inert gas]-containing gas in the step (1) is selected such that an inert gas reduction line connecting a point corresponding to a combustible gas concentration in the mixed gas to be produced (provided that only amounts of the combustion assisting gas and the combustible gas are considered, and an amount(s) of the other component(s) is not considered in the case where the other component(s) is present in addition to the combustion assisting gas and the combustible gas) and an apex of 0% of the combustion assisting gas, 100% of the inert gas, and 0% of the combustible gas does not intersect with the explosion range of the combustion assisting gas-inert gas-combustible gas system.

Invention 5. The method according to any one of inventions 1 to 4 **characterized in that** the combustible gas in the step (2) is a first combustible gas, and the method further comprises (3) the step of mixing another combustible gas as a second combustible gas with the [combustion assisting gas, inert gas, and combustible gas]-containing gas obtained in the step (2) so as to prepare a [combustion assisting gas, inert gas, the first combustible gas, and the second combustible gas]-containing gas, whereby the method produces in place of the mixed gas comprising the combustion assisting gas and the combustible gas, the [combustion assisting gas, inert gas, the first combustible gas, and the second combustible gas]-containing gas.

Invention 6. The method according to invention 5 **characterized in that** a composition of the [combustion assisting gas, inert gas, first combustible gas, and second combustible gas]-containing gas obtained by the step (3) (provided that only amounts of the combustion assisting gas, the first combustible gas, and the second combustible gas are considered, and amounts of the inert gas and the other component(s) (if any) are not considered) is outside the explosion range of the combustion assisting gas-first combustible gas-second combustible gas system.

Invention 7. The method according to invention 6 **characterized in that** an amount of the second combustible gas to be mixed in the step (3) is selected such that a second combustible gas addition line connecting a point corresponding to the first combustible gas concentration in the [combustion assisting gas, inert gas and first combustible gas]-containing gas with the reduced amount of the inert gas (provided that only the amounts of the combustion assisting gas and the first combustible gas are considered, and other component(s) is not considered in the case where the other component(s) is present in addition to the combustion assisting gas and the first combustible gas) and an apex of 0% of the combustion assisting gas, 0% of the first combustible gas, 100% of the second combustible gas does not intersect with the explosion range of the combustion assisting gas-first combustible gas-second combustible gas system in the equilateral triangle coordinate graph indicating such explosion range.

Invention 8. The method according to any one of inventions 1 to 7 **characterized in that** the [combustion assisting gas and inert gas]-containing gas is a mixed gas obtained by a preliminary mixing step wherein the combustion assisting gas and the inter gas is mixed beforehand.

Invention 9. The method according to any one of inventions 1 to 8 **characterized in that** the inert gas is water vapor or carbon dioxide.

Invention 10. The method according to invention 9 **characterized in that** the inert gas is water vapor, and the inert gas reduction step (2) is carried out by a condensation step wherein water vapor is condensed.

Invention 11. The method according to any one of inventions 1 to 10 **characterized in that** the combustion assisting gas is oxygen or air.

Invention 12. The method according to any one of inventions 1 to 11 **characterized in that** the combustible gas or the first combustible gas is an olefin.

Invention 13. The method according to any one of inventions 5 to 12 **characterized in that** the second combustible gas is hydrogen.

Invention 14. The method according to invention 12 or 13 **characterized in that** the olefin is $\alpha$-olefin.

Invention 15. The method according to invention 14 **characterized in that** the $\alpha$-olefin is propylene.

Invention 16. An apparatus for producing a mixed gas which comprises a combustion assisting gas and a combustible gas **characterized in that** the apparatus comprises:

(A) a mixing apparatus for mixing the combustion assisting gas, the inert gas and a combustible gas which comprises:

a means for supplying a [combustion assisting gas and inert gas]-containing gas which comprises the

combustion assisting gas and the inert gas;
a means for supplying the comestible gas; and
a means for mixing those gases; and

(B) an apparatus for reducing an amount of the inert gas of a [combustion assisting gas, inert gas, and combustible gas]-containing gas which is supplied from the mixing apparatus (A).

Invention 17. An apparatus for producing a mixed gas which comprises a combustion assisting gas, a first combustible gas, and a second combustible gas **characterized in that** the apparatus comprises:

(A') a mixing apparatus (A) according to invention 16 wherein the means for supplying the comestible gas is a means for supplying the first combustible gas,
(B') an apparatus for reducing an amount of the inert gas of a [combustion assisting gas, inert gas, and first combustible gas]-containing gas which is supplied from the mixing apparatus (A'); and
(C) a second mixing apparatus comprising:

a means for supplying the [combustion assisting gas, inert gas and first combustible gas]-containing gas which is supplied from the apparatus (B') for reducing an amount of the inert gas;
a means for supplying the second combustible gas which is different from the first combustible gas; and
a means for mixing the [combustion assisting gas, inert gas and first combustible gas]-containing gas and the second combustible gas.

Invention 18. The apparatus according to invention 17 **characterized in that** the means for supplying the [combustion assisting gas, and inert gas]-containing gas of the mixing apparatus (A') comprises a means for supplying from a preliminary mixing apparatus which comprises:

a means for supplying the combustion assisting gas;
a means for supplying the inert gas;
a means for mixing the combustion assisting gas and the inert gas.

Invention 19. The apparatus according to any one of inventions 16 to 18 **characterized in that** the apparatus for reducing (B) or (B') is a cooling and/or compressing apparatus which cools and/or compresses a gas which is supplied to the apparatus.
Invention 20. The apparatus according to any one of inventions 16 to 19 **characterized in that** the inert gas is water vapor, and the inert gas reduction apparatus is a condensation apparatus of the inert gas.
Invention 21. The apparatus according to invention 20 **characterized in that** the condensation apparatus comprises a heat exchanger and a gas-liquid separation apparatus;
the heat exchanger comprises a coolant flow passage and a gas flow passage wherein
the [combustion assisting gas, water vapor and combustible gas or first combustible gas]-containing gas flows through the gas flow passage so that water vapor is condensed,
a gas-liquid mixture obtained after the condensation is supplied to the gas-liquid separation apparatus so as to be divided into a gas and a liquid, and
said liquid is subjected to a lower pressure and supplied to the coolant flow passage of the heat exchanger.
Invention 22. The apparatus according to any one of inventions 16 to 21 **characterized in that** the combustion assisting gas is oxygen.
Invention 23. The apparatus according to any one of inventions 17 to 22 **characterized in that** the first combustible gas is an olefin.
Invention 24. The apparatus to any one of inventions 17 to 23 **characterized in that** the second combustible gas is hydrogen.
Invention 25. An apparatus for epoxidizing an olefin comprising a reactor and the apparatus for producing the mixed gas according to any one of inventions 17 to 21, which apparatus is **characterized in that**
the combustion assisting gas is oxygen, the inert gas is water vapor, the first combustible gas is the olefin, and the second combustible gas is hydrogen,
the reactor and the apparatus for producing the mixed gas are connected such that the [oxygen, water vapor, olefin, and hydrogen]-containing gas obtained by the apparatus for producing the mixed gas is supplied to the reactor; and the reactor produces an epoxy compound which corresponds to the olefin.
Invention 26. The apparatus according to invention 25 **characterized in that** the reactor has a function to circulate a gas phase and/or a liquid phase of the reactor.

Invention 27. The apparatus according to invention 26 **characterized in that** the reactor has a function to externally circulate its gas phase by means of a compressor, and also a function to supply the [oxygen, water vapor, olefin, and hydrogen]-containing gas to the reactor when it is added to the gas phase which is externally circulated.

Invention 28. The apparatus according to invention 27 **characterized in that** the function is attained by an ejector, into which the [oxygen, water vapor, olefin, and hydrogen]-containing gas is sucked so that the gas is supplied to the reactor.

Invention 29. The apparatus according to invention 25 **characterized in that** the reactor has a function to internally circulate a gas phase of the reactor into a liquid phase of the reactor.

Invention 30. The apparatus according to invention 29 **characterized in that** the reactor comprises a self-sucking type gas-liquid contact stirrer.

Invention 31. The apparatus according to invention 30 **characterized in that** the reactor comprises a stirrer which entraining a part of the gas phase of the reactor into the liquid phase of the reactor.

Invention 32. A method for producing an epoxy compound of an olefin, **characterized in that** the method comprises the steps of:

(a) obtaining, by the method for producing the mixed gas according to any one of inventions 5 to 10 wherein the combustion assisting gas is oxygen and the first combustible gas is an olefin, an [oxygen, water vapor, olefin, and hydrogen]-containing gas;
(b) supplying the obtained [oxygen, water vapor, olefin, and hydrogen]-containing gas to a reactor; and
(c) reacting oxygen, the olefin, and hydrogen in the presence of a catalyst in a liquid phase of the reactor so as to prepare the epoxy compound corresponding to the olefin.

Invention 33. The method according to invention 32 **characterized in that** an α-olefin is used as the olefin.

Invention 34. The method according to invention 33 **characterized in that** propylene is used as the α-olefin.

Invention 35. The method according to any one of inventions 32 to 34 **characterized in that** a liquid phase which comprises

a quinoid compound or a dihydro-form of a quinoid compound is used as the liquid phase.

Invention 36. The method according to any one of inventions 32 to 34 **characterized in that** a liquid phase which comprises acetonitrile is used as the liquid phase.

Invention 37. The method according to any one of inventions 32 to 36 **characterized in that** a catalyst comprising titanosilicate and a noble metal is used as the catalyst.

Invention 38. The method according to invention 37 **characterized in that** the titanosilicate is a crystalline titanosilicate having MWW structure or a lamellar titanosilicate.

Invention 39. The method according to invention 37 or 38 **characterized in that** the noble metal is palladium.

Invention 40. The method according to any one of inventions 32 to 39 **characterized in that** the step (c) comprises the step of circulating a gas phase and/or a liquid phase of the reactor.

Invention 41. The method according to invention 40 **characterized in that** the step for circulating externally circulates the gas phase of the reactor by means of a compressor while supplying the [oxygen, water vapor, olefin, and hydrogen]-containing gas to an externally circulating stream or the gas phase.

Invention 42. The method according to invention 40 **characterized in that** the step for circulating externally circulates the liquid phase of the reactor through an ejector while the [oxygen, water vapor, olefin, and hydrogen]-containing gas is sucked by the ejector so as to supply the gas to the reactor.

Invention 43. The method according to invention 40 **characterized in that** the step for circulating internally circulates the gas phase to the liquid phase.

Invention 44. The method according to invention 43 **characterized in that** the step for circulating internally circulates the gas phase to the liquid phase is an internally circulating step by means of a self-sucking type gas-liquid contact stirrer.

Invention 45. The method according to invention 43 **characterized in that** the step for circulating internally circulates the gas phase to the liquid phase uses a stirrer which has a function to entrain a part of the gas phase of the reaction into the liquid phase of the reaction.

## Claims

1. A method for producing a mixed gas which comprises a combustion assisting gas and a combustible gas **characterized in that** the method comprises the steps of:

(1) mixing a [combustion assisting gas and inert gas]-containing gas which comprises the combustion assisting

gas and an inert gas with the comestible gas so as to prepare a [combustion assisting gas, inert gas, and combustible gas]-containing gas which comprises the combustion assisting gas, the inert gas, and the combustible gas; and
(2) reducing an amount of the inert gas contained in the [combustion assisting gas, inert gas, and combustible gas]-containing gas.

**2.** The method according to claim 1 **characterized in that** the step (1) adjusts a composition and/or an amount of the [combustion assisting gas and inert gas]-containing gas as well as an amount of the combustible gas to be mixed and mixes these gases in such a manner that a composition of the [combustion assisting gas, inert gas, and combustible gas]-containing gas which is obtained after the mixing is positioned outside the explosion range of a combustion assisting gas-inert gas-combustible gas system.

**3.** The method according to claim 2 **characterized in that** a ratio between the combustion assisting gas and the inert gas contained in the [combustion assisting gas and inert gas]-containing gas to be mixed with the combustible gas in the step (1) is selected such that, in an equilateral triangle coordinate graph indicating the explosion range of the combustion assisting gas-inert gas-combustible gas system, a combustible gas addition line connecting a point corresponding to a combustion assisting gas concentration in the [combustion assisting gas and inert gas]-containing gas (provided that only amounts of the combustion assisting gas and the inert gas are considered, and an amount (s) of the other component(s) is not considered when such other component(s) is present in addition to the combustion assisting gas and the inert gas) and an apex of 0% of the combustion assisting gas, 0% of the inert gas, and 100% of the combustible gas does not intersect the explosion range of the combustion assisting gas-inert gas-combustible gas system.

**4.** The method according to claim 3 **characterized in that** an amount of the combustible gas to be mixed with the [combustion assisting gas and inert gas]-containing gas in the step (1) is selected such that an inert gas reduction line connecting a point corresponding to a combustible gas concentration in the mixed gas to be produced (provided that only amounts of the combustion assisting gas and the combustible gas are considered, and an amount(s) of the other component(s) is not considered in the case where the other component(s) is present in addition to the combustion assisting gas and the combustible gas) and an apex of 0% of the combustion assisting gas, 100% of the inert gas, and 0% of the combustible gas does not intersect with the explosion range of the combustion assisting gas-inert gas-combustible gas system.

**5.** The method according to any one of claims 1 to 4
**characterized in that** the combustible gas in the step (2) is a first combustible gas, and the method further comprises (3) the step of mixing another combustible gas as a second combustible gas with the [combustion assisting gas, inert gas, and combustible gas]-containing gas obtained in the step (2) so as to prepare a [combustion assisting gas, inert gas, the first combustible gas, and the second combustible gas]-containing gas, whereby the method produces ,in place of the mixed gas comprising the combustion assisting gas and the combustible gas, the [combustion assisting gas, inert gas, the first combustible gas, and the second combustible gas]-containing gas.

**6.** The method according to claim 5 **characterized in that** a composition of the [combustion assisting gas, inert gas, first combustible gas, and second combustible gas]-containing gas obtained by the step (3) (provided that only amounts of the combustion assisting gas, the first combustible gas, and the second combustible gas are considered, and amounts of the inert gas and the other component(s) (if any) are not considered) is outside the explosion range of the combustion assisting gas-first combustible gas-second combustible gas system.

**7.** The method according to claim 6 **characterized in that** an amount of the second combustible gas to be mixed in the step (3) is selected such that a second combustible gas addition line connecting a point corresponding to the first combustible gas concentration in the [combustion assisting gas, inert gas and first combustible gas]-containing gas with the reduced amount of the inert gas (provided that only the amounts of the combustion assisting gas and the first combustible gas are considered, and other component(s) is not considered in the case where the other component(s) is present in addition to the combustion assisting gas and the first combustible gas) and an apex of 0% of the combustion assisting gas, 0% of the first combustible gas, 100% of the second combustible gas does not intersect with the explosion range of the combustion assisting gas-first combustible gas-second combustible gas system in the equilateral triangle coordinate graph indicating such explosion range.

**8.** The method according to any one of claims 1 to 7
**characterized in that** the [combustion assisting gas and inert gas]-containing gas is a mixed gas obtained by a

preliminary mixing step wherein the combustion assisting gas and the inter gas is mixed beforehand.

**9.** The method according to any one of claims 1 to 8
**characterized in that** the inert gas is water vapor or carbon dioxide.

**10.** The method according to claim 9 **characterized in that** the inert gas is water vapor, and the inert gas reduction step (2) is carried out by a condensation step wherein water vapor is condensed.

**11.** The method according to any one of claims 1 to 10 **characterized in that** the combustion assisting gas is oxygen or air.

**12.** The method according to any one of claims 1 to 11 **characterized in that** the combustible gas or the first combustible gas is an olefin.

**13.** The method according to any one of claims 5 to 12 **characterized in that** the second combustible gas is hydrogen.

**14.** The method according to claim 12 or 13
**characterized in that** the olefin is $\alpha$-olefin.

**15.** The method according to claim 14 **characterized in that** the $\alpha$-olefin is propylene.

**16.** An apparatus for producing a mixed gas which comprises a combustion assisting gas and a combustible gas **characterized in that** the apparatus comprises:

(A) a mixing apparatus for mixing the combustion assisting gas, the inert gas and a combustible gas which comprises:

a means for supplying a [combustion assisting gas and inert gas]-containing gas which comprises the combustion assisting gas and the inert gas;
a means for supplying the comestible gas; and
a means for mixing those gases; and

(B) an apparatus for reducing an amount of the inter inert gas of a [combustion assisting gas, inert gas, and combustible gas]-containing gas which is supplied from the mixing apparatus (A).

**17.** An apparatus for producing a mixed gas which comprises a combustion assisting gas, a first combustible gas, and a second combustible gas **characterized in that** the apparatus comprises:

(A') a mixing apparatus (A) according to claim 16 wherein the means for supplying the comestible gas is a means for supplying the first combustible gas,
(B') an apparatus for reducing an amount of the inter inert gas of a [combustion assisting gas, inert gas, and first combustible gas]-containing gas which is supplied from the mixing apparatus (A'); and.
(C) a second mixing apparatus comprising:

a means for supplying the [combustion assisting gas, inert gas and first combustible gas]-containing gas which is supplied from the apparatus (B') for reducing an amount of the inter inert gas;
a means for supplying the second combustible gas which is different from the first combustible gas; and
a means for mixing the [combustion assisting gas, inert gas and first combustible gas]-containing gas and the second combustible gas.

**18.** The apparatus according to claim 17
**characterized in that** the means for supplying the [combustion assisting gas, and inert gas]-containing gas of the mixing apparatus (A') comprises a means for supplying from a preliminary mixing apparatus which comprises:

a means for supplying the combustion assisting gas;
a means for supplying the inert gas;
a means for mixing the combustion assisting gas and the inert gas.

**19.** The apparatus according to any one of claims 16 to 18 **characterized in that** the apparatus for reducing (B) or (B')

is a cooling and/or compressing apparatus which cools and/or compresses a gas which is supplied to the apparatus.

**20.** The apparatus according to any one of claims 16 to 19 **characterized in that** the inert gas is water vapor, and the inert gas reduction apparatus is a condensation apparatus of the inert gas.

**21.** The apparatus according to claim 20
**characterized in that** the condensation apparatus comprises a heat exchanger and a gas-liquid separation apparatus;
the heat exchanger comprises a coolant flow passage and a gas flow passage wherein
the [combustion assisting gas, water vapor and combustible gas or first combustible gas]-containing gas flows through the gas flow passage so that water vapor is condensed,
a gas-liquid mixture obtained after the condensation is supplied to the gas-liquid separation apparatus so as to be divided into a gas and a liquid, and
said liquid is subjected to a lower pressure and supplied to the coolant flow passage of the heat exchanger.

**22.** The apparatus according to any one of claims 16 to 21 **characterized in that** the combustion assisting gas is oxygen.

**23.** The apparatus according to any one of claims 17 to 22 **characterized in that** the first combustible gas is an olefin.

**24.** The apparatus to any one of claims 17 to 23
**characterized in that** the second combustible gas is hydrogen.

**25.** An apparatus for epoxidizing an olefin comprising a reactor and the apparatus for producing the mixed gas according to any one of claims 17 to 21, which apparatus is **characterized in that**
the combustion assisting gas is oxygen, the inert gas is water vapor, the first combustible gas is the olefin, and the second combustible gas is hydrogen,
the reactor and the apparatus for producing the mixed gas are connected such that the [oxygen, water vapor, olefin, and hydrogen]-containing gas obtained by the apparatus for producing the mixed gas is supplied to the reactor; and the reactor produces an epoxy compound which corresponds to the olefin.

**26.** The apparatus according to claim 25
**characterized in that** the reactor has a function to circulate a gas phase and/or a liquid phase of the reactor.

**27.** The apparatus according to claim 26
**characterized in that** the reactor has a function to externally circulate its gas phase by means of a compressor, and also a function to supply the [oxygen, water vapor, olefin, and hydrogen]-containing gas to the reactor when it is added to the gas phase which is externally circulated.

**28.** The apparatus according to claim 27
**characterized in that** the function is attained by an ejector, into which the [oxygen, water vapor, olefin, and hydrogen]-containing gas is sucked so that the gas is supplied to the reactor.

**29.** The apparatus according to claim 25
**characterized in that** the reactor has a function to internally circulate a gas phase of the reactor into a liquid phase of the reactor.

**30.** The apparatus according to claim 29
**characterized in that** the reactor comprises a self-sucking type gas-liquid contact stirrer.

**31.** The apparatus according to claim 30
**characterized in that** the reactor comprises a stirrer which entraining a part of the gas phase of the reactor into the liquid phase of the reactor.

**32.** A method for producing an epoxy compound of an olefin, **characterized in that** the method comprises the steps of:

(a) obtaining, by the method for producing the mixed gas according to any one of claims 5 to 10 wherein the combustion assisting gas is oxygen and the first combustible gas is an olefin, an [oxygen, water vapor, olefin, and hydrogen]-containing gas;

(b) supplying the obtained [oxygen, water vapor, olefin, and hydrogen]-containing gas to a reactor; and
(c) reacting oxygen, the olefin, and hydrogen in the presence of a catalyst in a liquid phase of the reactor so as to prepare the epoxy compound corresponding to the olefin.

**33.** The method according to claim 32 **characterized in that** an $\alpha$-olefin is used as the olefin.

**34.** The method according to claim 33 **characterized in that** propylene is used as the $\alpha$-olefin.

**35.** The method according to any one of claims 32 to 34 **characterized in that** a liquid phase which comprises a quinoid compound or a dihydro-form of a quinoid compound is used as the liquid phase.

**36.** The method according to any one of claims 32 to 34 **characterized in that** a liquid phase which comprises acetonitrile is used as the liquid phase.

**37.** The method according to any one of claims 32 to 36 **characterized in that** a catalyst comprising titanosilicate and a noble metal is used as the catalyst.

**38.** The method according to claim 37 **characterized in that** the titanosilicate is a crystalline titanosilicate having MWW structure or a lamellar titanosilicate.

**39.** The method according to claim 37 or 38
**characterized in that** the noble metal is palladium.

**40.** The method according to any one of claims 32 to 39 **characterized in that** the step (c) comprises the step of circulating a gas phase and/or a liquid phase of the reactor.

**41.** The method according to claim 40 **characterized in that** the step for circulating externally circulates the gas phase of the reactor by means of a compressor while supplying the [oxygen, water vapor, olefin, and hydrogen]-containing gas to an externally circulating stream or the gas phase.

**42.** The method according to claim 40 **characterized in that** the step for circulating externally circulates the liquid phase of the reactor through an ejector while the [oxygen, water vapor, olefin, and hydrogen]-containing gas is sucked by the ejector so as to supply the gas to the reactor.

**43.** The method according to claim 40 **characterized in that** the step for circulating internally circulates the gas phase to the liquid phase.

**44.** The method according to claim 43 **characterized in that** the step for circulating internally circulates the gas phase to the liquid phase is an internally circulating step by means of a self-sucking type gas-liquid contact stirrer.

**45.** The method according to claim 43 **characterized in that** the step for circulating internally circulates the gas phase to the liquid phase uses a stirrer which has a function to entrain a part of the gas phase of the reaction into the liquid phase of the reaction.

[Fig. 1]

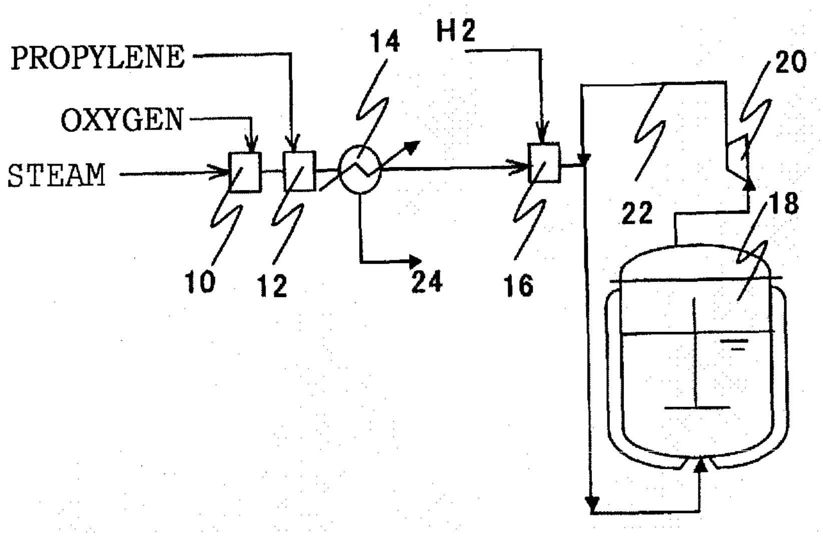
PROPYLENE
OXYGEN
STEAM
14
H2
20
22
18
10
12
24
16

[Fig. 2]

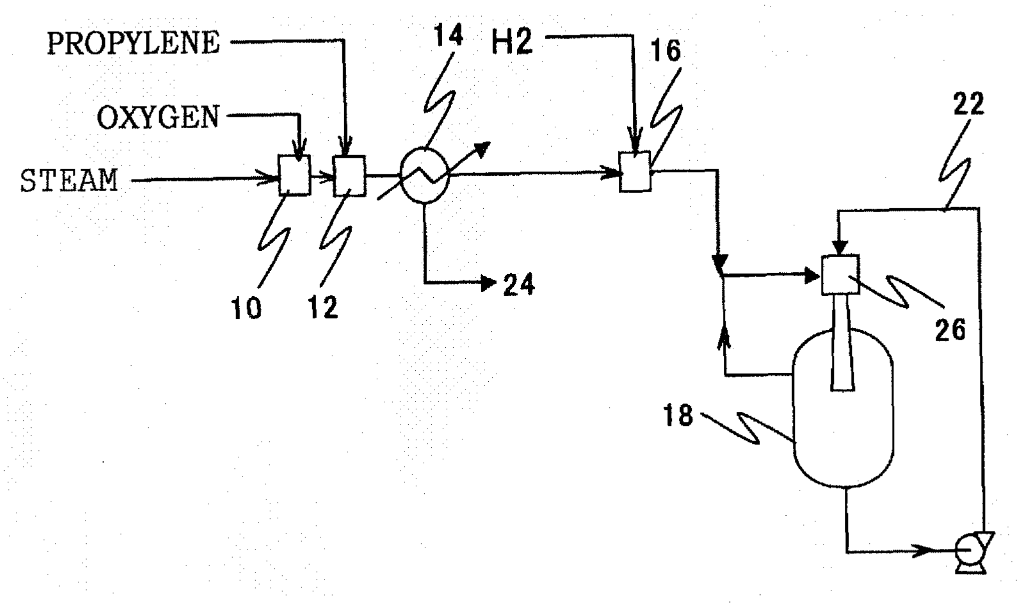
PROPYLENE
OXYGEN
STEAM
14
H2
16
22
10
12
24
26
18

[Fig. 3]

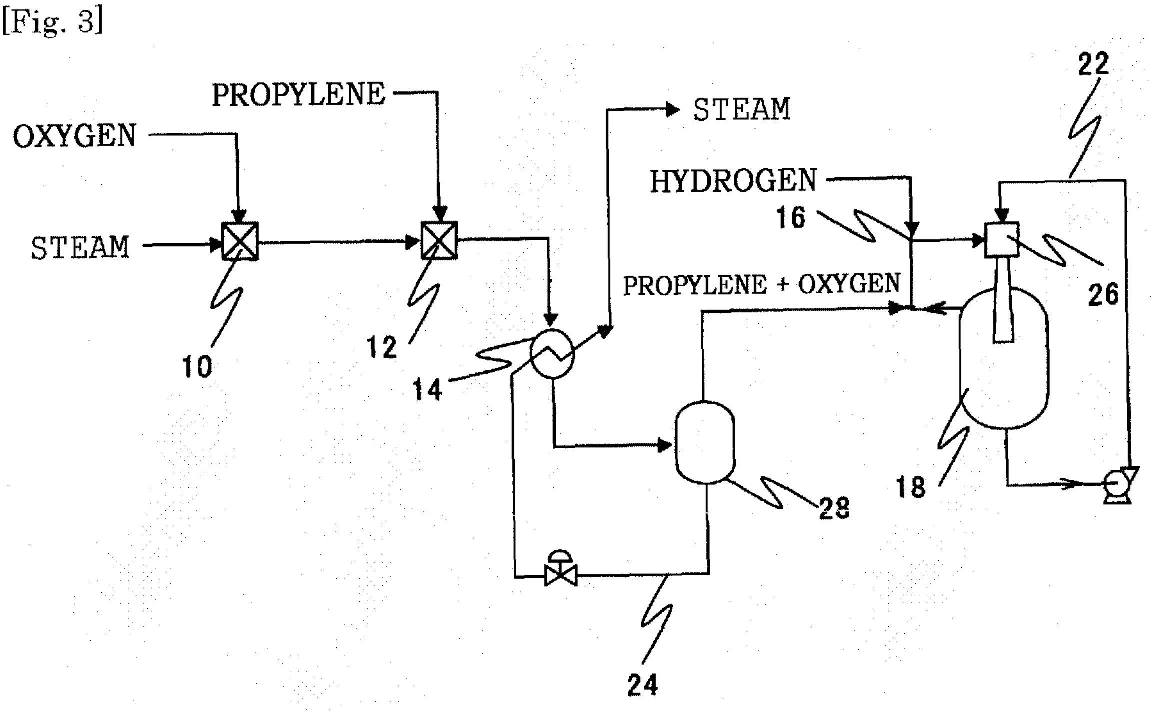
PROPYLENE
OXYGEN
STEAM
STEAM
HYDROGEN
PROPYLENE + OXYGEN
10
12
14
16
18
22
24
26
28

[Fig. 4]

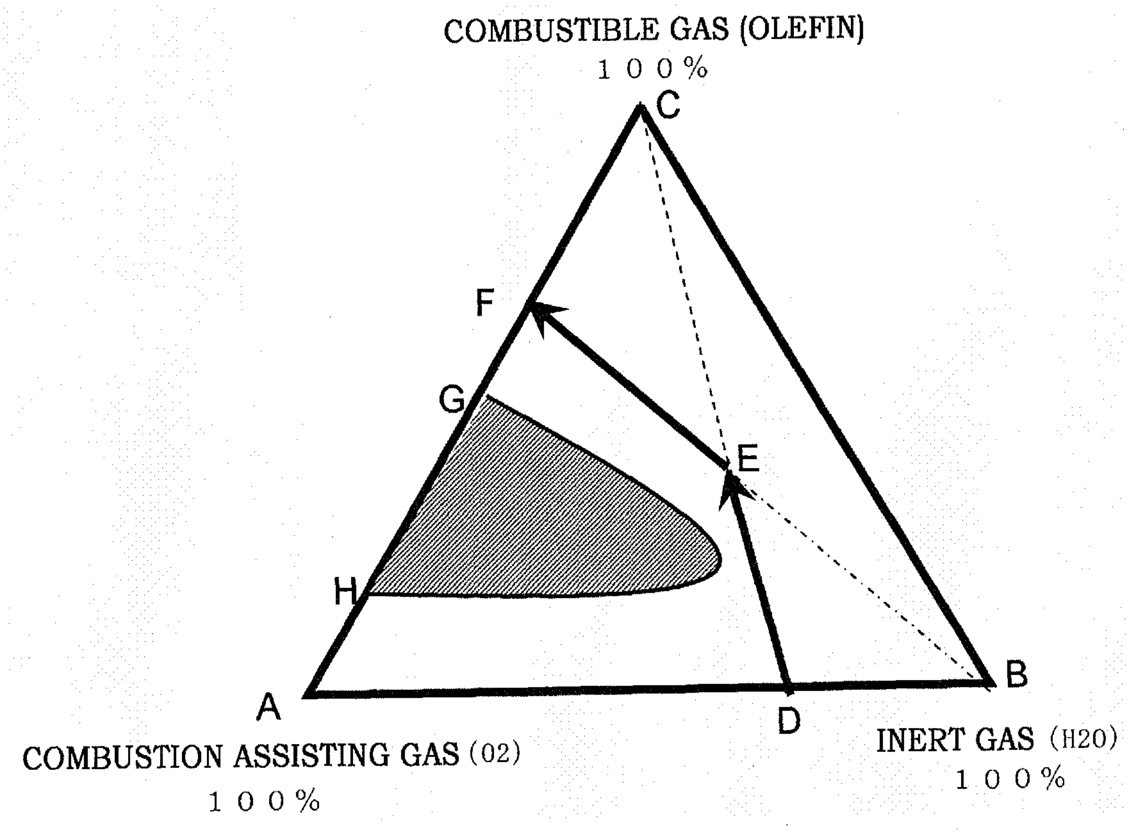
COMBUSTIBLE GAS (OLEFIN)
100%
C
F
G
E
H
A
D
B
COMBUSTION ASSISTING GAS (O2)
100%
INERT GAS (H2O)
100%

[Fig. 5]

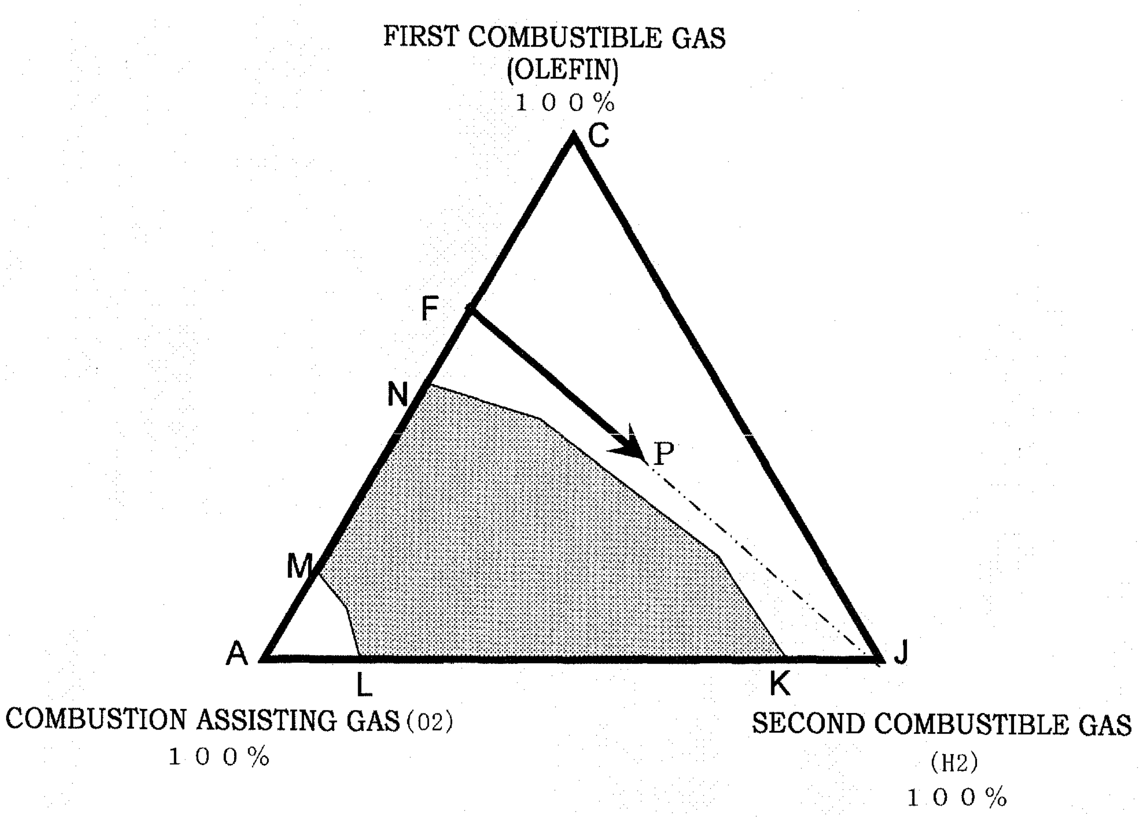
FIRST COMBUSTIBLE GAS
(OLEFIN)
100%
C
F
N
P
M
A
L
K
J
COMBUSTION ASSISTING GAS (O2)
100%
SECOND COMBUSTIBLE GAS
(H2)
100%

[Fig. 6]

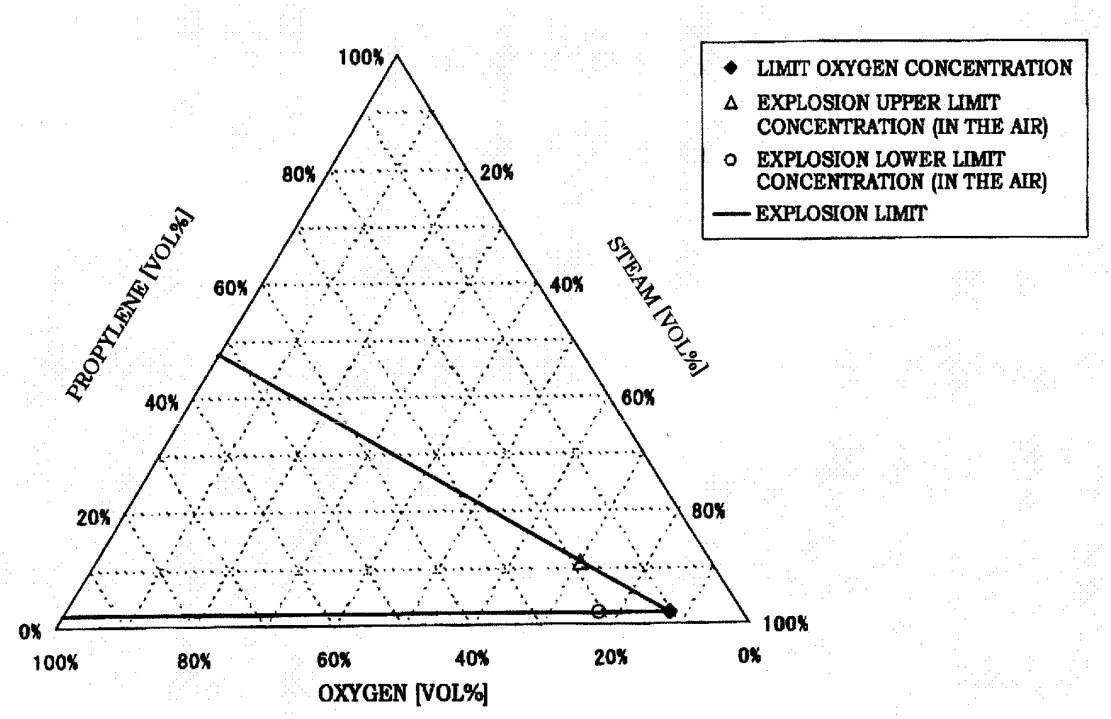
LIMIT OXYGEN CONCENTRATION
EXPLOSION UPPER LIMIT CONCENTRATION (IN THE AIR)
EXPLOSION LOWER LIMIT CONCENTRATION (IN THE AIR)
EXPLOSION LIMIT
PROPYLENE [VOL%]
STEAM [VOL%]
OXYGEN [VOL%]
100%
80%
60%
40%
20%
0%

[Fig. 7]

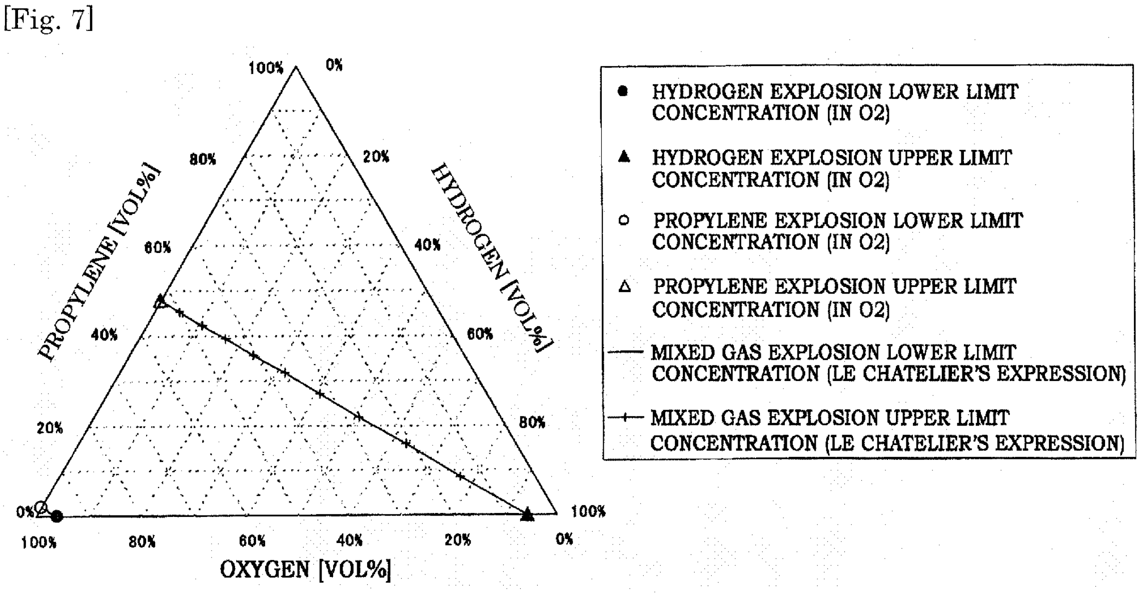
HYDROGEN EXPLOSION LOWER LIMIT CONCENTRATION (IN O2)
HYDROGEN EXPLOSION UPPER LIMIT CONCENTRATION (IN O2)
PROPYLENE EXPLOSION LOWER LIMIT CONCENTRATION (IN O2)
PROPYLENE EXPLOSION UPPER LIMIT CONCENTRATION (IN O2)
MIXED GAS EXPLOSION LOWER LIMIT CONCENTRATION (LE CHATELIER'S EXPRESSION)
MIXED GAS EXPLOSION UPPER LIMIT CONCENTRATION (LE CHATELIER'S EXPRESSION)
PROPYLENE [VOL%]
HYDROGEN [VOL%]
OXYGEN [VOL%]
100%
80%
60%
40%
20%
0%

**INTERNATIONAL SEARCH REPORT**

International application No.
PCT/JP2008/062325

A. CLASSIFICATION OF SUBJECT MATTER
*C07D301/08*(2006.01)i, *B01J29/89*(2006.01)i, *C07D303/04*(2006.01)i, *C07B61/00*(2006.01)n

According to International Patent Classification (IPC) or to both national classification and IPC

B. FIELDS SEARCHED

Minimum documentation searched (classification system followed by classification symbols)
C07D301/08, B01J29/89, C07D303/04, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 59-29629 A (Mitsubishi Petrochemical Co., Ltd.), 16 February, 1984 (16.02.84), Particularly, Claims; page 1, lower right column, the lowest line to page 2, upper left column, 1st paragraph; page 2, lower right column, 3rd line from the bottom to page 3, upper part; Fig. 2 (Family: none) | 1-4,8-12, 14-25<br>13,26-31 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 October, 2008 (01.10.08) | 14 October, 2008 (14.10.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/062325

C (Continuation). DOCUMENTS CONSIDERED TO BE RELEVANT

| Category* | Citation of document, with indication, where appropriate, of the relevant passages | Relevant to claim No. |
|---|---|---|
| Y | JP 8-245494 A (BASF AG.), 24 September, 1996 (24.09.96), & US 5705684 A & EP 731077 A2 & DE 19508558 A & DE 19525504 A & DE 59603316 D & CA 2171417 A & CZ 9600733 A & ES 2139266 T & SG 40844 A & TW 328534 A & CN 1143068 A | 13 |
| Y | WO 2007/074760 A1 (Sumitomo Chemical Co., Ltd.), 05 July, 2007 (05.07.07), All references; particularly, Par. Nos. [0004] to [0019], [0036] to [0038]; examples (Family: none) | 26-31 |
| Y | JP 2003-510314 A (ARCO CHEMICAL TECHNOLOGY, L.P.), 18 March, 2003 (18.03.03), Particularly, Par. Nos. [0019], [0021] to [0022]; examples & WO 01/23370 A1 & US 6063942 A & US 6063942 A & AU 6625400 A & BR 14290 A & CA 2379563 A & CN 1376151 A | 26-31 |
| Y | JP 7-8789 A (Kawaken Fine Chemicals Co., Ltd.), 13 January, 1995 (13.01.95), Par. No. [0002] (Family: none) | 27-28 |
| Y | JP 7-47262 A (BIAZZI S.A.), 21 February, 1995 (21.02.95), & US 5478535 A & EP 633060 A1 & DE 69406726 D & CH 686117 A | 29-31 |

Form PCT/ISA/210 (continuation of second sheet) (April 2007)

**INTERNATIONAL SEARCH REPORT**

International application No.
PCT/JP2008/062325

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 5-7
because they relate to parts of the international application that do not comply with the prescribed requirements to such an extent that no meaningful international search can be carried out, specifically:
Definitions as "a first combustible gas" and "a second combustible gas" only specify that the gases are combustible, but do not clearly define combinations of gases.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006517522 A
- JP 2005514364 A
- JP 2003327425 A


### Non-patent literature cited in the description


- **Tadao Yoshida ; Tei Taigyoku.** Safety Technology of Chemical Substances. Tokyo Progress System, 01 November 1996, 112-113
- New Safety Engineering Handbook. Colona Publishing Company, 20 July 1999, 140-150
- New Safety Engineering Handbook. Colona Publishing Company, 20 July 1999, 134
- Separate Volute of Chemical Industry. Disaster Prevention Engineering. Kabushiki Kaisha Kagaku Kogyo-Sha, 15 June 1966, 183
- *Zeolites,* 1995, vol. 15, 236-242
- *Journal of Catalysis,* 2001, vol. 199, 41-47
- *Chemistry Letters,* 2000, 774-775
- *Zeolites,* 1995, vol. 15, 519-525
- *Journal of Catalysis,* 1991, 130
- Angewandte Chemie. 2001, vol. 43, 236-240
- *Microporous Materials,* 1997, vol. 10, 259-271
- *Chemical Communications,* 1996, 145-146
- *Chemistry of Materials,* 2002, vol. 14, 1657-1664
- *Microporous and Mesoporous Materials,* 2002, vol. 52, 11-18